# EUROPEAN PATENT APPLICATION

(11) **EP 4 268 811 A1**
(43) Date of publication of application: **01.11.2023**
(21) Application number: 21911629.0
(22) Date of filing: 27.12.2021
(51) Int. Cl.: A61K 31/165, A61P 29/00, A61P 17/00, A61P 37/08

(54) **NOVEL DERIVATIVE COMPOUND HAVING BIPHENYL GROUP INTRODUCED INTO AMINO-ALKANOIC ACID AND ANTI-INFLAMMATORY COMPOSITION COMPRISING SAME**

(30) Priority: 25.12.2020 KR 20200184007
(71) Applicant: Amtixbio Co., Ltd., Seoul 01411 (KR)
(72) Inventor: LEE, Jongseung, Seoul 01738 (KR); KIM, Dahee, Hanam-si, Gyeonggi-do 12925 (KR); SEO, Kyoung Hee, Hanam-si, Gyeonggi-do 12925 (KR)
(74) Representative: Bringer IP
(86) International application number: PCT/KR2021/019974
(87) International publication number: WO 2022/139564

(57) **Abstract**

The present invention relates to a novel aminoalkanoic acid derivative compound into which a biphenyl group is introduced and an anti-inflammatory composition comprising the same. The purpose of this invention is to provide a pharmaceutical composition for preventing or treating fibrotic diseases, inflammatory diseases, autoimmune diseases, and/or allergic diseases containing a novel aminoalkanoic acid derivative compound into which a biphenyl group a biphenyl, its salt and/or a solvent as active ingredient.

## Description

### [Technical Field]

The present invention relates to a derivative compound in which a biphenyl group is introduced into an aminoalkanoic acid, a stereoisomer thereof, or a pharmaceutically acceptable salt thereof, and an anti-inflammatory pharmaceutical composition comprising the same as an active ingredient.

### [Background Art]

Cytokines play essential roles in the immune system, including cell-mediated immunity and allergic reactions. Cytokines are classified into proinflammatory and anti-inflammatory cytokines. They are mainly produced by T lymphocytes, which possess antigen-specific receptors on their cell surfaces, allowing them to recognize external pathogens. T lymphocytes are categorized based on surface molecules as CD4 and CD8 cells. The T lymphocytes expressing CD4, known as helper T cells, generate the highest levels of cytokines. Helper T cells are further classified into two types: Th1, which produces Th1-type cytokines, and Th2, which produces Th2-type cytokines. Th1-type cytokines primarily induce inflammatory responses to maintain immune reactions against intracellular foreign pathogens to remove them, with interferon-gamma being the major Th1 cytokine. Th2-type cytokines, on the other hand, include interleukin-4, interleukin-5, interleukin-6, interleukin-13, interleukin-10, etc., which are associated with promoting IgE and eosinophilic responses in atopic conditions.

Interleukin-6 (IL-6) is a cytokine that is generated early in inflammatory responses and has diverse biological functions, including acute-phase reactions, inflammation, hematopoiesis, bone metabolism, and induction of cancer progression, etc. Human IL-6 consists of 183 amino acids (aa) and shares 41% amino acid sequence identity with mouse and rat IL-6. It is secreted by T cells or macrophages to induce inflammatory responses. Due to its function in inducing inflammation and autoimmune reactions, anti-IL-6 drugs are being developed as potential therapies for various diseases, including rheumatoid arthritis and cancer. Further, IL-6 is associated with various biological phenomena such as synovial inflammation, immune reactions, and hematopoiesis. IL-6 forms a hexameric complex by binding to IL-6 receptors (IL-6R) and glycoprotein 130 (gp130). The intracellular signaling pathway involves Janus kinase (JAK) and signal transducer and activator of transcription (STAT) pathways. Pharmacological inhibitors of IL-6 signaling interfere with the binding of IL-6 to IL-6R, either targeting the cytokine itself or the receptor. IL-6 is associated with various diseases, including rheumatoid arthritis, systemic juvenile idiopathic arthritis (JIA), Castleman disease, giant cell arteritis, Takayasu arteritis, cytokine release syndrome, etc. Thus, anti-IL-6R drugs can be utilized to improve symptoms such as fever, fatigue, pain, joint destruction, and anemia, etc.. Additionally, IL-6 is known as a major contributor to cytokine storms, characterized by excessive cytokine release.

Thymic stromal lymphopoietin (TSLP) is produced in response to stimuli such as viruses, bacteria, fungi, protease allergens, inflammatory cytokines, chemicals, and allergens, etc., by skin keratinocytes or epithelial cells of the lungs and intestines. In performing its biological functions, TSLP first binds to its receptor, TSLP receptor (TSLPR), and subsequently forms a heterodimeric complex with interleukin-7 receptor subunit alpha (IL7Rα), activating a signaling cascade. This heterodimeric complex polarizes dendritic cells to induce type 2 inflammatory responses and acts as a master regulator for Th2 immune responses on the surfaces of the skin, gut, and airways. Upon formation of the TSLPR and IL-7Ra complex, cross-phosphorylation of JAK1 and JAK2 occurs, followed by the induction of signal transducer and activator of transcription 5 (STAT5).

TSLP has been found to be secreted not only by epithelial cells and keratinocytes but also by other cell types such as mast cells, airway smooth muscle, fibroblasts, or dendritic cells, regulating immune responses. TSLP expression in the skin has been reported to be associated with various allergic conditions (Steven F. Ziegler et al., J Allergy Clin Immunol, 2012; 130: 845-52). TSLP has also been detected in bronchial lavage fluid of asthma patients, and an association has been reported between increased TSLP expression in sensory nerves and airway epithelial cells in the pathogenesis of the disease. It has been revealed that allergenic inflammatory responses are aggravated and asthma development is promoted upon antigen exposure due to TSLP secretion from skin cells (Juan et al., Journal of Investigative Dermatology, 2012). TSLP receptor (TSLPR) is mainly expressed in hematopoietic cells and particularly abundant in dendritic cells. TSLP is involved in communication between tissue cells and immune cells as well as the interaction of innate and adaptive immune systems. Therefore, TSLP and its signaling have been implicated in conditions such as atopic dermatitis (AD), psoriasis, asthma, allergic rhinitis (AR), and eosinophilic esophagitis (EoE). Additionally, TSLP signaling has been reported to be associated with other immune-mediated disorders such as solid tumors (e.g., breast, colon, and pancreatic cancer) and hematological malignancies (e.g., B-cell acute lymphoblastic leukemia (B-ALL)). Moreover, TSLP is an important pruritogen. Tezepelumab, a human monoclonal antibody targeting TSLP, is being developed as a therapeutic agent for asthma and atopic dermatitis (TSLP: from allergy to cancer, Jonathan Corren & Steven F. Ziegler, Nature Immunology volume 20, 1603 - 1609 (2019)).

TSLP has also been associated with fibrosis (Evidence for a functional TSLP signaling axis in fibrotic lung disease, Arnab Datta, et al., J Immunol. 2013 Nov 1; 191(9)). Fibrosis of tissues can severely impair their function. For example, liver fibrosis progresses to liver cirrhosis and hepatic cancer, and fibrotic tissues are commonly observed in chronic pancreatitis and pancreatic cancer. Fibrotic diseases include liver fibrosis, chronic hepatitis, cirrhosis, hepatic cancer, chemotherapy-associated steatohepatitis (CASH), lung fibrosis, renal fibrosis, renal failure, pancreatic fibrosis, chronic pancreatitis, and pancreatic cancer, etc.

### [Disclosure]

### [Technical Problem]

The present invention is to provide an anti-inflammatory pharmaceutical composition comprising, as a pharmacologically active ingredient, a novel derivative compound aminoalkanoic acid derivative containing a biphenyl group.

In addition, the invention is to provide a pharmaceutical composition that inhibits IL-6 signaling (IL6/IL-6R/JAK), comprising the said aminoalkanoic acid derivative compound as a pharmacologically active ingredient.

In addition, the invention aims to provide an anti-inflammatory composition that inhibits TSLP signaling (TSLP/TSLPR/IL-7Rα), comprising the said aminoalkanoic acid derivative compound as a pharmacologically active ingredient.

Furthermore, the present invention is to provide a pharmaceutical composition for the prevention and/or treatment of inflammation, immune disorders, fibrotic diseases, and/or cancer, which comprises the said aminoalkanoic acid derivative compound as a pharmacologically active ingredient.

Furthermore, the present invention is to provide a pharmaceutical composition for the prevention and/or treatment of pruritus (itching), which comprises the said aminoalkanoic acid derivative compound as a pharmacologically active ingredient.

Furthermore, the present invention is to provide a pharmaceutical composition for the prevention and/or treatment of allergic disorders, which comprises the said aminoalkanoic acid derivative compound as a pharmacologically active ingredient.

Furthermore, the present invention is to provide a method for treating cancer, inflammatory diseases, fibrotic diseases, pruritus, allergies, and/or immune disorders by administering a pharmaceutical composition comprising the said aminoalkanoic acid derivative compound as a pharmacologically active ingredient into a subject in need.

### [Description]

### [Technical problem to be solved]

To achieve the above objectives, the present invention provides a novel aminoalkane derivative containing a biphenyl group, represented by the following chemical formula 1, or its pharmaceutically acceptable salts and/or solvates: 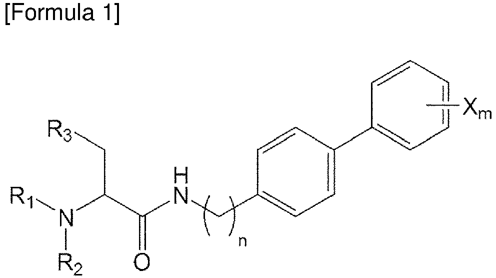 wherein, n is 0, 1 or 2,
R₁ and R₂ are each independently the same as or different from each other, and are each independently H or C₁₋₆ alkyl,
R₃ is C₁₋₆ alkyl, and
X is m substituents (m is an integer from 1 to 5) which are the same as or different from each other, selected from the group consisting of a halogen group, a halogenated C₁₋₆ alkyl group and a halogenated C₁₋₆ alkoxy group.

In addition, the compound represented by the Formula 1 is a compound having no limitation on a 3D arrangement structure of a substituent attached to a chiral carbon, and may include all structurally available enantiomers or optical isomers. In particular, the compound represented by the Formula 1 may be provided in the form of a (R) or (S) isomer thereof alone or a mixture thereof, for example, a racemic mixture or racemate thereof, but not limited thereto.

The present invention may include not only the said Formula 1 or a pharmaceutically acceptable salt thereof, but also a solvate or hydrate presenting the same effects, which can be prepared therefrom within the scope of the present invention.

The compound of the present invention is based on an aminoalkanoic acid and may be a derivative in which a biphenyl group is introduced into the aminoalkanoic acid.

For example, the aminoalkanoic acid may be an α-amino acid derivative containing a C₂₋₄ straight hydrocarbon chain in the side chain, for example, α-aminobutyric acid, norvaline or norleucine.

As used herein, the term "α-aminobutyric acid (AABA)" refers to a compound represented by the following Formula 6, which has an IUPAC name of 2-aminobutyric acid, and is a non-proteinogenic α-amino acid of formula C₄H₉NO₂, which is also known to be homoalanine in biochemistry. The α-aminobutyric acid includes a C₂ straight hydrocarbon chain in the side chain, which contains additional C₁ comparing to alanine.

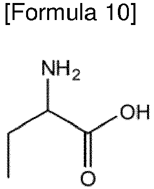

As used herein, the term "norvaline (Nva)" refers to a compound represented by the following Formula 7, which has an IUPAC name of 2-aminopentanoic acid, and is a watersoluble amino acid that is an isomer of valine, which is a branched chain amino acid (BCAA) of formula CH₃(CH₂)₂CH(NH₂)CO₂H.

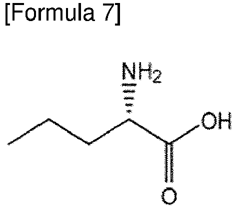

As used herein, the term "norleucine (Nle)" refer to a compound represented by the following Formula 8, which has an IUPAC name of 2-aminohexanoic acid, and is an amino acid having the formula CH₃(CH₂)₃CH(NH₂)CO₂H.

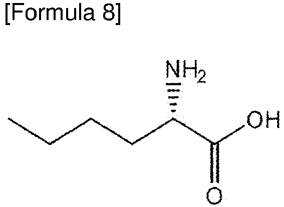

For example, the compound of the present invention may be a compound in which R₁ and R₂ are each independently H or methyl, ethyl, n-propyl, isopropyl, cyclopropyl, n-butyl, isobutyl, cyclobutyl, n-pentyl, cyclopentyl, n-hexyl or cyclohexyl, and R₃ is methyl, ethyl, n-propyl, isopropyl, cyclopropyl, n-butyl, isobutyl, sec-butyl or tert-butyl.

Furthermore, the compound of the present invention may be a compound in which R₁ and R₂ are each independently H or methyl, and R₃ is methyl, ethyl, or n-propyl, but not limited thereto.

For example, X in the compound of the present invention may be any one or two identical or different substituents selected from the group consisting of fluoro, chloro, trifluoromethyl and trifluoromethoxy. For example, the substituent may be one, or two or more which are the same as each other or differently selected.

For example, X in the compound of the present invention may be fluoro, chloro, trifluoromethyl or trifluoromethoxy, and in particular, X may be p-fluoro, m-fluoro, p,m-difluoro, p-chloro, m-chloro, p,m-dichloro, p-trifluoromethyl or p-trifluoromethoxy, but not limited thereto.

Specifically, the compound of the present invention may be
1) 2-amino-N-(3',4'-dichloro-[1,1'-biphenyl]-4-yl)butanamide;
2) 2-amino-N-(3',4'-dichloro-[1,1'-biphenyl]-4-yl)pentanamide;
3) 2-amino-N-(3',4'-dichloro-[1,1'-biphenyl]-4-yl)hexanamide;
4) 2-amino-N-(4'-(trifluoromethoxy)-[1,1'-biphenyl]-4-yl)pentanamide;
5) N-(3',4'-dichloro-[1,1'-biphenyl]-4-yl)-2-(methylamino)butanamide;
6) N-(3',4'-dichloro-[1,1'-biphenyl]-4-yl)-2-(methylamino)pentanamide;
7) N-(3',4'-dichloro-[1,1'-biphenyl]-4-yl)-2-(methylamino)hexanamide;
8) N-(3',4'-dichloro-[1,1'-biphenyl]-4-yl)-2-(dimethylamino)pentanamide;
9) 2-amino-N-((3',4'-dichloro-[1,1'-biphenyl]-4-yl)methyl)pentanamide;
10) 2-amino-N-((3',4'-dichloro-[1,1'-biphenyl]-4-yl)methyl)hexanamide;
11) 2-amino-N-((4'-(trifluoromethyl)-[1,1'-biphenyl]-4-yl)methyl)pentanamide;
12) 2-amino-N-((4'-(trifluoromethyl)-[1,1'-biphenyl]-4-yl)methyl)hexanamide;
13) 2-amino-N-((4'-(trifluoromethoxy)-[1,1'-biphenyl]-4-yl)methyl)pentanamide;
14) 2-amino-N-((4'-(trifluoromethoxy)-[1,1'-biphenyl]-4-yl)methyl)hexanamide;
15) N-((3',4'-dichloro-[1,1'-biphenyl]-4-yl)methyl)-2-(methylamino)pentanamide;
16) 2-(methylamino)-N-((4'-(trifuloromethyl)-[1,1'-biphenyl]-4-yl)methyl)pentanamide;
17) N-((3',4'-dichloro-[1,1'-biphenyl]-4-yl)methyl)-2-(dimethylamino)pentanamide;
18) 2-amino-N-(2-(3',4'-dichloro-[1,1'-biphenyl]-4-yl)ethyl)butanamide;
19) 2-amino-N-(2-(3',4'-dichloro-[1,1'-biphenyl]-4-yl)ethyl)pentanamide;
20) 2-amino-N-(2-(3',4'-dichloro-[1,1'-biphenyl]-4-yl)ethyl)hexanamide;
21) 2-amino-N-(2-(4'-(trifluoromethyl)-[1,1'-biphenyl]-4-yl)ethyl)butanamide;
22) 2-amino-N-(2-(4'-(trifluoromethyl)-[1,1'-biphenyl]-4-yl)ethyl)pentanamide;
23) 2-amino-N-(2-(4'-(trifluoromethyl)-[1,1'-biphenyl]-4-yl)ethyl)hexanamide;
24) 2-amino-N-(2-(4'-(trifluoromethoxy)-[1,1'-biphenyl]-4-yl)ethyl)butanamide;
25) 2-amino-N-(2-(4'-(trifluoromethoxy)-[1,1'-biphenyl]-4-yl)ethyl)pentanamide;
26) 2-amino-N-(2-(4'-(trifluoromethoxy)-[1,1'-biphenyl]-4-yl)ethyl)hexanamide;
27) 2-amino-N-(2-(3',4'-difluoro-[1,1'-biphenyl]-4-yl)ethyl)pentanamide;
28) N-(2-(3',4'-dichloro-[1,1'-biphenyl]-4-yl)ethyl)-2-(methylamino)butanamide;
29) N-(2-(3',4'-dichloro-[1,1'-biphenyl]-4-yl)ethyl)-2-(methylamino)pentanamide;
30) N-(2-(3',4'-dichloro-[1,1'-biphenyl]-4-yl)ethyl)-2-(methylamino)hexanamide;
31) 2-(methylamino)-N-(2-(4'-(trifluoromethyl)-[1,1'-biphenyl]-4-yl)ethyl)butanamide;
32) 2-(methylamino)-N-(2-(4'-(trifluoromethyl)-[1,1'-biphenyl]-4-yl)ethyl)pentanamide;
33) 2-(methylamino)-N-(2-(4'-(trifluoromethyl)-[1,1'-biphenyl]-4-yl)ethyl)hexanamide;
34) 2-(methylamino)-N-(2-(4'-(trifluoromethoxy)-[1,1'-biphenyl]-4-yl)ethyl)butanamide;
35) 2-(methylamino)-N-(2-(4'-(trifluoromethoxy)-[1,1'-biphenyl]-4-yl)ethyl)pentanamide;
36) 2-(methylamino)-N-(2-(4'-(trifluoromethoxy)-[1,1'-biphenyl]-4-yl)ethyl)hexanamide; or
37) N-(2-(3',4'-dichloro-[1,1'-biphenyl]-4-yl)ethyl)-2-(dimethylamino)pentanamide;
but is not limited thereto.

In the present invention, the term "chiral" refers to a molecule that possesses a characteristic that cannot be superimposed onto its mirror image partner, while "achiral" refers to a molecule that can be superimposed onto its mirror image partner. "Enantiomer" refers to a pair of stereoisomers that are non-superimposable mirror images of each other. The stereochemical definitions and conventions used in the present specification generally follow the references [S. P. Parker, Ed., McGraw-Hill Dictionary of Chemical Terms (1984) McGraw-Hill Book Company, New York] and [Eliel, E. and Wilen, S., "Stereochemistry of Organic Compounds", John Wiley & Sons, Inc., New York, 1994].

The compound of the present invention may be in the form of a pharmaceutically acceptable salt. As a salt, an acid addition salt formed by a pharmaceutically acceptable free acid may be used. As used herein, the term "pharmaceutically acceptable salt" refers to an organic or inorganic addition salt of the compound represented by Formula 1 that is tolerated and sufficiently non-toxic to be used for patients at any concentration exhibiting pharmacological effects of the compound.

The acid addition salt is prepared by typical methods, for example, dissolving the compound in an excess aqueous acid solution, and precipitating the obtained salt using a water-miscible organic solvent, for example, methanol, ethanol, acetone or acetonitrile. The same molar amount of compound and acid or alcohol (for example, glycol monomethyl ether) in water are heated, and then the resulting mixture may be evaporated and dried, or the precipitated salt may be suction filtered.

In this case, as the free acid, organic acids and inorganic acids may be used. Further, as the inorganic acids, hydrochloric acid, phosphoric acid, sulfuric acid, nitric acid, tartaric acid, and the like may be used, and as the organic acids, methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like may be used, but the free acid is not limited thereto.

Further, a pharmaceutically acceptable metal salt may be prepared using a base. An alkali metal salt or an alkaline earth metal salt is obtained, for example, by dissolving the compound in an excessive amount of an alkali metal hydroxide or alkaline earth metal hydroxide solution, filtering a non-dissolved compound salt, and then evaporating and drying a filtrate. In this case, as the metal salt, it is pharmaceutically preferable to prepare particularly, sodium, potassium, or calcium salts, but the metal salt is not limited thereto. In addition, a silver salt corresponding thereto may be obtained by reacting an alkali metal or alkaline earth metal salt with a suitable silver salt (for example, silver nitrate).

The pharmaceutically acceptable salt of the compound of the present invention includes a salt of an acidic or basic group that may be present in the compound of Formula 1, unless otherwise indicated. For example, the pharmaceutically acceptable salt may comprise sodium, calcium, potassium salts and the like of a hydroxyl group. The examples of other pharmaceutically acceptable salts of an amino group include hydrobromide, sulfate, hydrogen sulfate, phosphate, hydrogen phosphate, dihydrogen phosphate, acetate, succinate, citrate, tartrate, lactate, mandelate, methanesulfonate (mesylate), p-toluenesulfonate (tosylate) salts, and the like, and may be prepared by a salt preparation method known in the art.

The salt of the compound of Formula 1 of the present invention is a pharmaceutically acceptable salt, and any salt is available without limitation as long as the salt shows a pharmacological activity equivalent to that of the compound of Formula 1. For example, a salt of the compound of Formula 1 presents antifungal activity.

As another aspect, the present invention provides a method for preparing a derivative compound, a stereoisomer thereof, or a pharmaceutically acceptable salt thereof in which a biphenyl group is introduced into the aminoalkanoic acid, the method including a first step of forming a peptide bond by reacting an aminoalkanoic acid derivative compound protected by a butoxycarbonyl (Boc) protecting group, which is represented by the following Formula 2, with a biphenyl derivative compound including a primary amine group, which is represented by the following Formula 3; and a second step of removing the Boc protecting group by reacting the compound obtained in the first step with an acid: 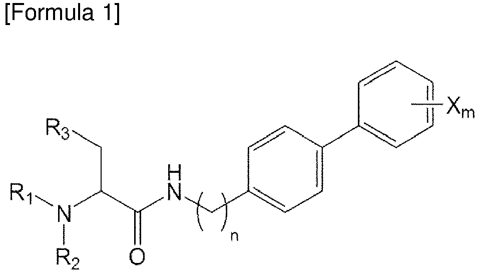 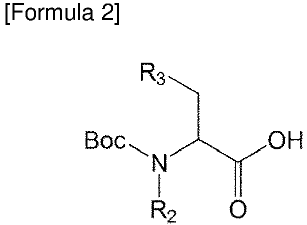 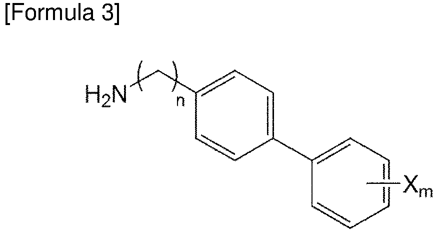
wherein, n is 0, 1 or 2,
R₁ and R₂ are each independently the same as or different from each other, and are each independently H or C₁₋₆ alkyl,
R₃ is C₁₋₆ alkyl, and
X is m substituents (m is an integer from 1 to 5) which are the same as or different from each other, selected from the group consisting of a halogen group, a halogenated C₁₋₇ alkyl group and a halogenated C₁₋₇ alkoxy group.

In the preparation method of the present invention, the aminoalkanoic acid derivative compound protected by the Boc protecting group, which is represented by Formula 2 may be prepared by reacting an amino acid derivative represented by the following Formula 4 with di-tert-butyl dicarbonate (also known as Boc anhydride): 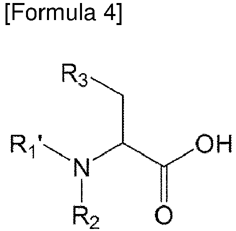
wherein, R'₁ and R₂ are each independently the same as or different from each other, and are each independently H or C₁₋₆ alkyl, and
R₃ is C₁₋₆ alkyl.

In this case, when R₂ of the finally prepared compound is an alkyl, a step of alkylating the compound by reacting the compound with a haloalkane in the presence of a base after the above reaction may be further performed. For example, the alkylation may be carried out by dissolving the compound represented by Formula 4 and a haloalkane compound corresponding 5 to 20 equivalents of the compound, for example, alkane iodide in an organic solvent, for example, tetrahydrofuran, adding sodium hydride as a base at a low temperature, for example, 0°C and then reacting the reactants at 15 to 30°C for 12 to 48 hours, but not limited thereto. An alkylation reaction of amines known in the art may be used without limitation or performed by being modified.

Meanwhile, in the preparation method of the present invention, a biphenyl derivative compound containing a primary amine group, which is represented by Formula 3 may be prepared by reacting a C₀₋₂ alkylamine derivative in which a halophenyl group at one end is substituted, which is represented by the following Formula 5 with di-tert-butyl dicarbonate to introduce a Boc protecting group into an amine group, reacting the resulting alkylamine derivative with a phenylboronic acid derivative represented by the following Formula 6, and then reacting the reactants with an acid to remove the Boc protecting group: 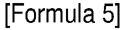 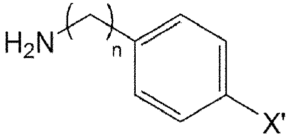 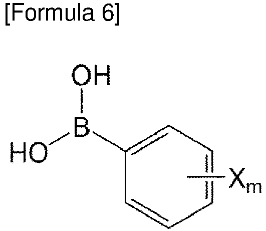
in the said formulae,
X' is a halogen, and
X is m substituents (m is an integer from 1 to 5) which are the same as or different from each other, selected from the group consisting of a halogen group, a halogenated C₁₋₇ alkyl group and a halogenated C₁₋₇ alkoxy group.

In this case, the reaction with the phenylboronic acid derivative may be achieved by a cross-coupling reaction using a metal catalyst in the presence of a base. For example, the reaction may be performed under basic conditions by a metal catalyst such as palladium or nickel. The metal catalyst may be a catalyst in which a phosphine ligand is bound to a metal. For example, the reaction may be a Suzuki-Miyaura cross-coupling reaction performed by Pd(PPh₃)₄ in the presence of Na₂CO₃, but is not limited thereto.

For example, in the preparation method of the present invention, the first step may be achieved by an anhydride coupling reaction performed in an organic solvent in the presence of N-methylmorpholine (NMM) or isobutyl chloroformate (IBCF). As the organic solvent, tetrahydrofuran may be used, but the organic solvent is not limited thereto.

For example, in the preparation method of the present invention, the second step to remove the Boc protecting group may be performed by carrying out a reaction with hydrochloric acid, but is not limited thereto. Furthermore, the preparation method of the present invention may further include a third step of forming a secondary amine by alkylating amine after the second step when each of R₁ and R₂ of the compound finally prepared is an alkyl. The amination may be performed by reacting with formaldehyde while supplying hydrogen gas in the presence of Pd/C as a reducing agent. For example, the reaction may be performed at 15 to 30°C for 6 to 24 hours, but is not limited thereto, and the alkylation reaction of amines known in the art may be used without limitation or performed by being modified.

In yet another aspect, the present invention provides a pharmaceutical composition for the prevention and/or treatment of inflammatory diseases, which comprises a compound of formula 1, containing a biphenyl group, its enantiomers, optical isomers, stereoisomers, diastereoisomers, tautomers, or their pharmaceutically acceptable salts, as pharmacologically active components. For example, the inflammatory diseases that can be prevented and/or treated with the pharmaceutical composition of the present invention may include atopic dermatitis, asthma, allergic rhinitis, allergic conjunctivitis, allergic dermatitis, psoriasis, inflammatory bowel disease, and food allergy, among others.

In yet another aspect, the present invention provides a pharmaceutical composition for the prevention and/or treatment of fibrotic diseases, which comprises a compound of formula 1, containing a biphenyl group, its enantiomers, optical isomers, stereoisomers, diastereoisomers, tautomers, or their pharmaceutically acceptable salts, as pharmacologically active components. For example, the fibrotic diseases that can be prevented and/or treated with the pharmaceutical composition of the present invention may include liver fibrosis, chronic hepatitis, cirrhosis, hepatic cancer, chemotherapy-associated steatohepatitis (CASH), nonalcoholic steatohepatitis (NASH), lung fibrosis, renal fibrosis, renal failure, pancreatic fibrosis, chronic pancreatitis, and pancreatic cancer, among others.

As used herein, the term "prevention" refers to any actions that suppress, inhibit or delay the onset, development or recurrence of any concerned disease by administering the said pharmaceutical composition. The term "treatment" refers to any actions in which the symptoms of any concerned disease are alleviated or beneficially improved by administering the said pharmaceutical composition.

The pharmaceutical composition according to the present invention may contain the compound represented by Formula 1, its stereoisomer, diastereomer, enantiomer, optical isomer, tautomeric isomer, or pharmaceutically acceptable salt thereof as an active ingredient, and it may further include a pharmaceutically acceptable carrier, diluent or excipient. For example, the pharmaceutical composition according to the present invention may be formulated and used in various forms such as an oral dosage form such as a powder, granules, a tablet, a capsule, a suspension, an emulsion, a syrup, or an aerosol, or an injection of a sterile injection solution by a conventional method according to each intended use, and may be administered orally or through various routes including intravenous, intraperitoneal, subcutaneous, rectal, topical administration and the like. Examples of a suitable carrier, diluent, or excipient that may be included in such a composition include lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oil, and the like. Further, the composition of the present invention may further include a filler, an anticoagulant, a lubricant, a wetting agent, a fragrance, an emulsifying agent, a preservative, and the like.

A solid preparation for oral administration includes a tablet, a pill, a powder, granules, a capsule, and the like, and the solid preparation is formulated by mixing at least one excipient, for example, starch, calcium carbonate, sucrose, lactose, gelatin, and the like with the composition. Further, in addition to a simple excipient, a lubricant such as magnesium stearate and talc may be used.

As a liquid preparation for oral administration, a suspension, a liquid for internal use, an emulsion, a syrup or the like may be used, and in addition to water and liquid paraffin, which are simple commonly used diluents, various excipients, for example, a wetting agent, a sweetener, a flavoring agent, a preserving agent or the like may be employed.

Examples of a preparation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like. As a base of the suppository, it is possible to use Witepsol^{®}, Macrogol, Tween 61, cacao butter, laurin fat, glycerogelatin, and the like. Meanwhile, the injection may include additives in the related art, such as a solubilizer, an isotonic agent, a suspending agent, an emulsifier, a stabilizer, and a preservative.

The composition of the present invention is administered in a pharmaceutically effective amount. The term "pharmaceutically effective amount" as used herein refers to an amount that is sufficient enough to treat diseases at a reasonable benefit/risk ratio applicable to medical treatment and does not cause side effects, and an effective dosage level may be determined according to various factors including patient's health status, type of diseases, severity of disease, activity of drugs, sensitivity to drugs, administration method, administration time, administration route and excretion rate, duration of treatment, and drugs used in combination or simultaneously, and other factors well known in the medical field. The composition of the present invention may be administered as an individual therapeutic agent or in combination with other therapeutic agents, may be administered sequentially or simultaneously with certain conventional therapeutic agents, and may be administered in a single dose or multiple doses. It is important to administer the composition in a minimum amount that can obtain the maximum effects without any side effects, in consideration of all the aforementioned factors, and this amount may be easily determined by those skilled in the art. For example, since the amount may be increased or decreased depending on the administration route, the severity of disease, gender, body weight, age, and the like, the dosage is not intended to limit the scope of the present invention in any way.

In one embodiment, the compound of the invention is preferably administered topically or orally. When administered topically, a therapeutically effective amount of a compound of the present invention may range from 0.00001 to about 20% (w/w), preferably 0.001 to 3%, although not limited thereto. When administered orally, the therapeutically effective amount of the compound of the present invention may be 0.01 to about 1,000 mg/kg, preferably 1 to about 300 mg/kg, but is not limited thereto. In the present invention, the subject to which the pharmaceutical composition of the present invention is administered is a human, but it may include an animal.

As used herein, the term "administration" refers to provision of a predetermined material to a patient by any appropriate method. With regard to the route of administration of the composition of the present invention, the composition of the present invention may be administered via any general route, which may reach a target tissue. The route of administration may be intraperitoneal administration, intravenous administration, intramuscular administration, subcutaneous administration, intradermal administration, oral administration, topical administration, intranasal administration, intrapulmonary administration, and rectal administration, but is not limited thereto. In addition, the pharmaceutical composition of the present invention may also be administered by any device which may allow an active material or ingredient to move to a target cell. Preferred administration modes and preparations are intravenous injection, subcutaneous injection, intradermal injection, intramuscular injection, drip injection and the like. An injectable preparation may be prepared using an aqueous solvent such as physiological saline and Ringer's solution, a non-aqueous solvent such as a vegetable oil, a higher fatty acid ester (for example, ethyl oleate, and the like), and an alcohol (for example, ethanol, benzyl alcohol, propylene glycol, glycerin, and the like), and may include a pharmaceutical carrier such as a stabilizer for preventing spoilage (for example, ascorbic acid, sodium bisulfite, sodium pyrosulfite, BHA, tocopherol, EDTA, and the like), an emulsifier, a buffer for pH control, and a preservative for inhibiting microbial growth (for example, phenylmercuric nitrate, thimerosal, benzalkonium chloride, phenol, cresol, benzyl alcohol, and the like).

The term "therapeutically effective amount" used in combination with the active ingredient in the present invention refers to an amount of a aminoalkanoic acid derivative compound in which a biphenyl group is introduced into aminoalkanoic acid, which is effective for preventing or treating a target disease, a stereoisomer thereof, or a pharmaceutically acceptable salt thereof.

### [Advantageous Effects]

The pharmaceutical composition of the present invention inhibits IL-6 signaling and/or TSLP/TSLPR/IL-7Rα signaling, thereby being able to be used for the development of a drug for effective prevention and treatment of inflammatory diseases, immune diseases, fibrotic diseases, allergies, itching and/or cancer.

### [Brief Description for Figures]

FIG.1 compares the IL-6 production in RAW264.7 cells when the cells were treated with LPS or the compound of the present invention at a concentration of 8 µg/ml.
FIG.2 compares the TNF-α production in RAW264.7 cells when the cells were treated with LPS or the compound of the present invention at a concentration of 8 µg/ml.
FIG.3 presents the results of evaluating the levels of TSLP and Interleukin-6 in HaCaT cells in response to the compound of the present invention.
FIG.4 and FIG.5 show whether or not the compound of the present invention inhibits the interaction between TSLP and TSLPR using HEK293T cells.
FIG.6 represents skin photographs showing psoriasis symptoms induced by Aldara cream.
FIG.7 shows a graph representing the skin index values for psoriasis symptoms induced by Aldara cream.
FIG.8 shows a graph illustrating the number of scratching events on the affected area for psoriasis symptoms induced by Aldara cream.
FIG.9 is a graph showing the results of efficacy evaluation at the RNA level for psoriasis symptoms induced by Aldera Cream.

### [Best Mode]

The reaction scheme for synthesizing the compounds of the present invention is as follows. In the reaction scheme below, the numbers that are not accompanied by English alphabets represent the compound numbers generated in the present invention. For example, 1 represents Compound 1:

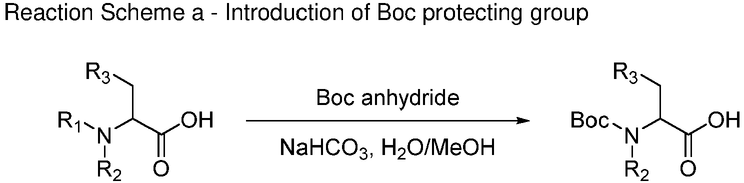

Norleucine (1.0 eq), Boc anhydride (1.5 eq), and sodium bicarbonate (1.5 eq) were dissolved in a 1:1 mixed solvent of distilled water and methanol and reacted at room temperature for 36 to 48 hours. After the mixture was concentrated in a vacuum state, the pH of an aqueous layer was adjusted to 2 with 1.0 M hydrochloric acid. Then, the moisture of an organic layer obtained by extraction with ethyl acetate was removed with sodium sulfate, and the solvent was evaporated in vacuum to obtain the title compound.

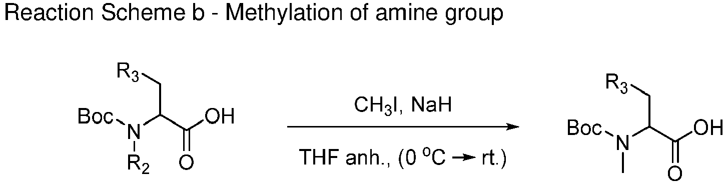

The compound (1.0 eq) obtained from Reaction Scheme a and iodomethane (10 eq) were dissolved in a tetrahydrofuran solvent, and sodium hydride (10 eq) was very slowly added dropwise thereto at 0°C. The reactants were reacted at room temperature for 24 hours. After the reaction was completed, the resulting product was diluted with an ether solvent, and distilled water was added thereto. The pH of an aqueous layer was adjusted to 2 with a 20% citric acid solution. Then, the moisture of an organic layer obtained by extraction with ethyl acetate was removed with sodium sulfate, and the solvent was evaporated in vacuum. The obtained residue was separated and purified by chromatography using silica gel to obtain the title compound.

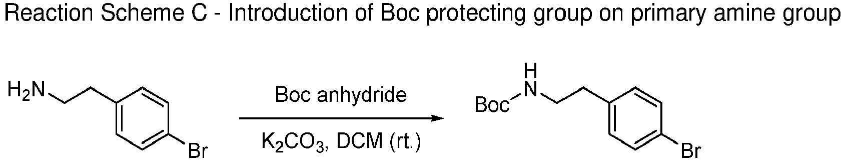

After 4-bromophenetylamine (1.0 eq) was dissolved in a methylene chloride solvent, potassium carbonate (1.5 eq) and Boc anhydride (1.05 eq) were added thereto, and the resulting mixture was reacted at room temperature for about 12 to 18 hours. The reaction mixture was diluted with methylene chloride and washed twice with distilled water. The organic layer was dried with sodium sulfate and then concentrated in vacuum. The obtained residue was washed with hexane and then evaporated in a vacuum state to obtain the title compound.

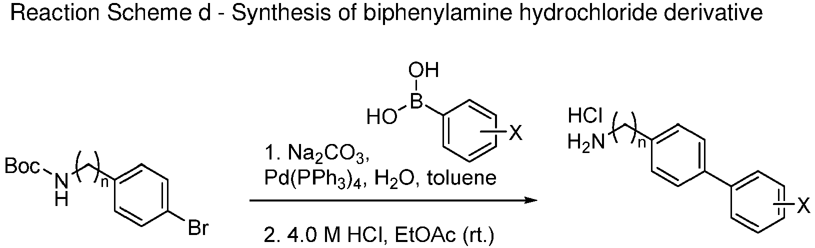

The compound obtained from Reaction Scheme c, tert-butyl (4-bromobenzyl)carbamate or tert-butyl (4-bromophenyl)carbamate (1.0 eq), benzene boronic acid (1.5 eq), sodium carbonate (5.0 eq), and tetrakis(triphenylphosphine)palladium (0.04 eq) were dissolved in a 2:1 to 2.5:1 mixed solvent of degassed toluene and distilled water, and reacted under reflux at a temperature of 140°C for 12 to 18 hours. After the reaction, the catalyst was removed by filtration through Celite, and the solvent was evaporated from the filtered organic layer in a vacuum state. The obtained residue was separated and purified by chromatography using silica gel. After the purified product was dissolved in an ethyl acetate solvent, the resulting solution was stirred at room temperature while adding 4.0 M hydrochloric acid (6.0 to 10.0 eq) thereto. The obtained white solid in the form of a salt was washed with ethyl acetate, and then completely dried in a vacuum state to obtain the title compound.

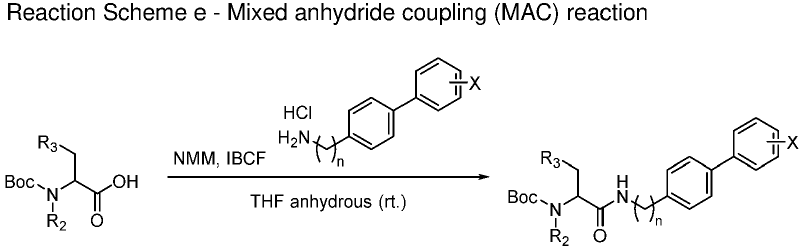

The compound synthesized according to Reaction Scheme a or the compound synthesized according to Reaction Scheme b (1.0 eq), and N-methylmorpholine (NMM, 2.5 to 2.8 eq) were put into a distilled tetrahydrofuran solvent and the resulting mixture was stirred for 15 minutes. Then isobutyl chloroformate (IBCF, 1.3 eq) was added thereto, and the resulting mixture was further stirred for 15 minutes, and then the compound (1.05 eq) obtained from Reaction Scheme d was added thereto. The reaction mixture was allowed to react at room temperature for about 3 to 5 hours. The mixture was filtered to evaporate the solvent in a vacuum state. The obtained residue was separated and purified by chromatography using silica gel to obtain the title compound.

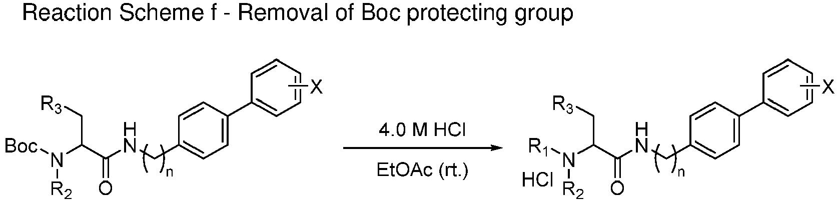

After the compound derivative (1.0 eq) obtained from Reaction Scheme e was dissolved in an ethyl acetate solvent, the resulting solution was stirred at room temperature while adding 4.0 M hydrochloric acid (6.0 to 10.0 eq) thereto. The obtained white solid in the form of a salt was washed with ethyl acetate, and then completely dried in a vacuum state to obtain the title compound.

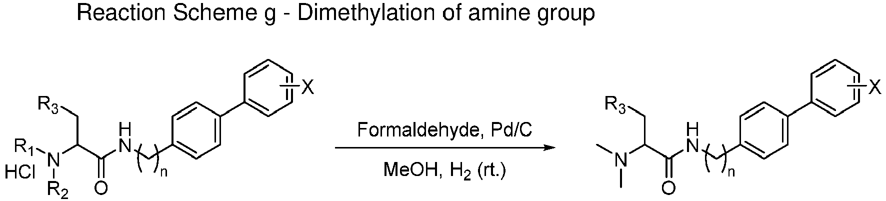

The compound (1.0 eq) obtained from Reaction Scheme f was dissolved in methanol, triethylamine (6.0 eq) was added thereto, and then formaldehyde (37% by weight solution, 1.0 to 2.5 eq) and a 10% palladium catalyst (0.1 to 0.5 eq) were sequentially added thereto. The reactants were reacted at room temperature for 18 hours. After the reaction, the catalyst was removed by filtration through Celite, and the filtered organic layer was evaporated in a vacuum state to obtain a white solid. The resulting product was recrystallized with methanol and diethyl ether to obtain the title compound.

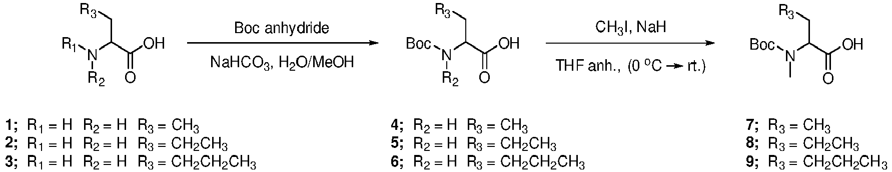

### [Embodiments of the Invention]

Hereinafter, the present invention will be described in more detail with reference to specific embodiments. However, these embodiments are intended to clarify the present invention in more detail, and it will be apparent to those skilled in the art that the scope of the present invention is not limited thereto.

### Preparation Examples

### Preparation Example 1: Preparation of (R)/(S)-2-((tert-butoxycarbonyl)amino)butanoic acid (4)

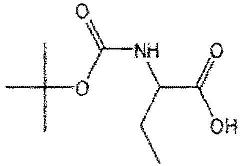

Compound 1 (2-aminobutanoic acid, 5.00 g, 48.5 mmol), Boc anhydride (19.9 mL, 72.7 mmol), and NaHCO₃ (6.11 g, 72.7 mmol) were reacted using Reaction Scheme a to synthesize Compound 4, (R)/(S)-2-((tert-butoxycarbonyl)amino)butanoic acid (8.25 g, 83%) in the form of a white powder.
R_{f} = 0.00 (DCM 9.5 : Methanol 0.5 and few drops of acetic acid);
¹H NMR (DMSO-*d₆*, 300 MHz) 12.40 (C(O)OH), 7.02 (d, *J* = 7.9 Hz, Boc-NH), 3.69-3.82 (m, Chiral-H), 1.48-1.72 (m, CH₂CH₃), 1.38 (s, Boc), 0.87 (t, J = 7.3 Hz, CH₂CH₃).

### Preparation Example 2: Preparation of (R)/(S)-2-((tert-butoxycarbonyl)amino)pentanoic acid (5)

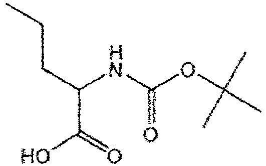

Compound 2 (2-aminopentanoic acid, 10.00 g, 25.6 mmol), Boc anhydride (35.1 mL, 128.0 mmol), and NaHCO₃ (10.8 g, 128.0 mmol) were reacted using Reaction Scheme a to synthesize Compound 5, (R)/(S)-2-((tert-butoxycarbonyl)amino)pentanoic acid (13.40 g, 83%) in the form of a white powder.
R_{f} = 0.85 (DCM 3 : Methanol 17);

¹H NMR(DMSO-*d₆*, 400 MHz) 12.40 (C(O)OH), 7.03 (d, *J* = 8.0 Hz, Boc-NH), 3.75-3.89 (m, Chiral-H), 1.50-1.65 (m, CH₂CH₂CH₃), 1.20-1.38 (m, CH₂CH₂CH₃, Boc), 0.85 (t, *J* = 7.4 Hz, CH₂CH₂CH₃).

### Preparation Example 3: Preparation of (R)/(S)-2-((tert-butoxycarbonyl)amino)hexanoic acid (6)

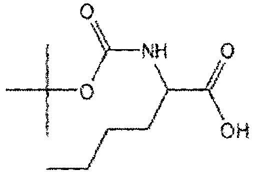

Compound 3 (2-aminohexanoic acid, 5.00 g, 38.1 mmol), Boc anhydride (15.7 mL, 57.2 mmol), and NaHCO₃ (4.80 g, 57.2 mmol) were reacted using Reaction Scheme a to synthesize Compound 6, (R)/(S)-2-((tert-butoxycarbonyl)amino)hexanoic acid (7.14 g, 81%) in the form of a white powder.
R_{f} = 0.40 (DCM 9 : Methanol 1);
¹H NMR (CDCl₃, 400 MHz) 10.26 (C(O)OH), 5.00 (d, *J* = 7.6 Hz, Boc-NH), 4.32-4.33 (m, Chiral-H), 1.63-1.87 (m, CH₂CH₂CH₂CH₃), 1.47 (s, Boc), 1.31-1.38 (m, CH₂CH₂CH₂CH₃), 0.93 (t, *J* = 7.0 Hz, CH₂CH₂CH₂CH₃).

### Preparation Example 4: Preparation of (R)/(S)-2-((tert-butoxycarbonyl)(methyl)amino)butanoic acid (7)

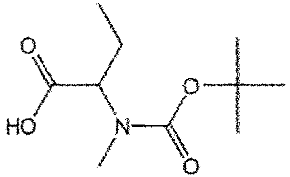

Compound 4 (3.00 g, 14.8 mmol), CH₃I (9.2 ml, 147.6 mmol), and NaH (3.54 g, 147.6 mmol) were reacted using Reaction Scheme b to synthesize Compound 7, (R)/(S)-2-((tert-butoxycarbonyl)(methyl)amino)butanoic acid (2.84 g, 88%) in the form of a yellow oil.
R_{f} = 0.45 (DCM 9 : Methanol 1 and few drops of acetic acid);
¹H NMR (DMSO-*d₆*, 300 MHz) 12.7 (C(O)OH), 4.14-4.43 (m, Chiral-H), 2.71 (s, NCH₃), 1.50-1.73 (m, CH₂CH₃, Boc), 0.79-0.87 (m, CH₂CH₃).

### Preparation Example 5: Preparation of (R)/(S)-2-((tert-butoxycarbonyl)(methyl)amino)pentanoic acid (8)

Compound 5 (1.50 g, 6.90 mmol), CH₃I (4.3 ml, 69.0 mmol), and NaH (1.66 g, 69.0 mmol) were reacted using Reaction Scheme b to synthesize Compound 8, (R)/(S)-2-((tert-butoxycarbonyl)(methyl)amino)pentanoic acid (1.34 g, 83%) in the form of a yellow oil.
R_{f} = 0.45 (DCM 9 : Methanol 1 and few drops of acetic acid);
¹H NMR (DMSO-*d₆*, 300 MHz) 12.7 (C(O)OH), 4.54-4.28 (m, Chiral-H), 2.70 (s, NCH₃), 1.79-1.64 (m, CH₂CH₂CH₃), 1.41-1.37 (m, CH₂CH₂CH₃, Boc), 1.37-1.29 (m, CH₂CH₂CH₃).

### Preparation Example 6: Preparation of (R)/(S)-2-((tert-butoxycarbonyl)(methyl)amino)hexanoic acid (9)

Compound 6 (3.00 g, 13.0 mmol), CH₃I (8.1 ml, 129.7 mmol), and NaH (5.19 g, 129.7 mmol) were reacted using Reaction Scheme b to synthesize Compound 9, (R)/(S)-2-((tert-butoxycarbonyl)(methyl)amino)hexanoic acid (3.18 g, 100%) in the form of a yellow oil.
R_{f} = 0.38 (DCM 9 : Methanol 1);
¹H NMR (CDCl₃, 400 MHz) 12.6 (C(O)OH), 4.25-4.52 (m, Chiral-H), 2.70 (s, NCH₃), 1.66-1.79 (m, CH₂CH₂CH₂CH₃), 1.18-1.40 (m, CH₂CH₂CH₂CH₃, Boc), 0.86-0.89 (m, CH₂CH₂CH₂CH₃).

### Preparation Example 7: Preparation of tert-butyl (4-bromophenethyl)carbamate (12)

4-bromophenethylamine (3.9 ml, 25.1 mmol), K₂CO₃ (5.21 g, 37.7 mmol), and Boc anhydride (7.2 ml, 26.4 mmol) were reacted using Reaction Scheme c to synthesize Compound 12, tert-butyl(4-bromophenethyl)carbamate (6.23 g, 83%) in the form of a white powder.
R_{f} = 0.36 (EtOAc 1 : n-hexane 5);
¹H NMR (DMSO-*d₆*, 400 MHz) 7.46 (d, *J* = 8.6 Hz, ArH), 7.15 (d, *J* = 8.2 Hz, ArH), 6.87 (s, NH), 3.09-3.14 (m, NHCH₂CH₂), 2.64-2.67 (m, NHCH₂CH₂), 1.35 (s, Boc).

### Preparation Example 8: Preparation of 3',4'-dichloro-[1,1'-biphenyl]-4-amine hydrochloride (13)

After a compound was obtained by reacting Compound 10 (tert-butyl 4-bromophenylcarbamate, 4.00 g, 14.7 mmol), 3,4-dichlorophenylboronic acid (3.37 g, 17.6 mmol), tetrakis(triphenylphosphine)palladium (0.68 g, 0.59 mmol), and Na₂CO₃ (7.80 g, 73.5 mmol) using Reaction Scheme d, a Boc group was removed using 4.0 M HCl (7.9 mL, 31.5 mmol in dioxane) to synthesize Compound 13, 3',4'-dichloro-[1,1'-biphenyl]-4-amine hydrochloride (1.27 g, 34%) in the form of a white powder.
R_{f} = 0.00 (EtOAc 9 : acetone 1);
¹H NMR (DMSO-*d₆*, 400 MHz) 9.94 (s, NH₃), 7.95 (d, *J* = 2.0 Hz, ArH), 7.40-7.80 (m, ArH), 7.39 (d, *J* = 8.5 Hz, ArH).

### Preparation Example 9: Preparation of 4'-(trifluoromethoxy)-[1,1'-biphenyl]-4-amine hydrochloride (14)

After a compound was obtained by reacting Compound 10 (3.99 g, 14.7 mmol), 4-(trifluoromethoxy)phenylboronic acid (7.77 g, 22.0 mmol), tetrakis(triphenylphosphine)palladium (0.68 g, 0.59 mmol), and Na₂CO₃ (7.77 g, 73.3 mmol) using Reaction Scheme d, a Boc group was removed using 4.0 M HCl (12.8 mL, 51.1 mmol in dioxane) to synthesize Compound 14, 4'-(trifluoromethoxy)-[1,1'-biphenyl]-4-amine hydrochloride (1.99 g, 48%) in the form of a white powder.
R_{f} = 0.00 (EtOAc 9 : acetone 1);
¹H NMR (DMSO-*d₆*, 400 MHz) 9.45 (bRs, NH₃), 7.77 (d, *J* = 8.7 Hz, ArH), 7.71 (d, *J* = 8.4 Hz, ArH), 7.45 (d, *J* = 8.4 Hz, ArH), 7.28 (d, *J* = 8.2 Hz, ArH).

### Preparation Example 10: Preparation of 2-(3',4'-dichloro-[1,1'-biphenyl]-4-yl)methan-1-amine hydrochloride (15)

After a compound was obtained by reacting Compound 11 (tert-butyl 4-bromobenzylcarbamate, 6.00 g, 21.0 mmol), 3,4-dichlorophenylboronic acid (4.80 g, 25.2 mmol), tetrakis(triphenylphosphine)palladium (0.97 g, 0.84 mmol), and Na₂CO₃ (111.1 g, 104.8 mmol) using Reaction Scheme d, a Boc group was removed using 4.0 M HCl (3.1 ml, 12.3 mmol in dioxane) to synthesize Compound 15, 2-(3',4'-dichloro-[1,1'-biphenyl]-4-yl)methan-1-amine hydrochloride (1.08 g, 17%) in the form of a white powder.
R_{f} = 0.00 (EtOAc 9 : acetone 1);
¹H NMR (DMSO-*d₆*, 400 MHz) 8.71 (s, NH₃), 7.97 (s, ArH), 7.63-7.83 (m, ArH), 4.07 (s, NH₃CH₂).

### Preparation Example 11: Preparation of (4'-(trifluoromethyl)-[1,1'-biphenyl]-4-yl)methanamine hydrochloride (16)

After a compound was obtained by reacting Compound 11 (6.00 g, 21.0 mmol), 4-(trifluoromethyl)phenylboronic acid (5.97 g, 31.5 mmol), tetrakis(triphenylphosphine)palladium (0.97 g, 0.84 mmol), and Na₂CO₃ (11.1 g, 104.8 mmol) using Reaction Scheme d, a Boc group was removed using 4.0 M HCl (17.9 ml, 71.7 mmol in dioxane) to synthesize Compound 16, (4'-(trifluoromethyl)-[1,1'-biphenyl]-4-yl)methanamine hydrochloride (1.08 g, 66%) in the form of a white powder.
R_{f} = 0.00 (EtOAc 9 : acetone 1);
¹H NMR (DMSO-*d₆*, 400 MHz) 8.49 (s, NH₃), 7.93 (d, *J* = 8.2 Hz, ArH), 7.83 (t, *J* = 9.0 Hz, ArH), 7.64 (d, *J* = 8.2 Hz, ArH), 4.09 (s, NH₃CH₂).

### Preparation Example 12: Preparation of (4'-(trifluoromethoxy)-[1,1'-biphenyl]-4-yl)methanamine hydrochloride (17)

After a compound was obtained by reacting Compound 11 (4.00 g, 14.0 mmol), 4-(trifluoromethoxy)phenylboronic acid (4.32 g, 21.0 mmol), tetrakis(triphenylphosphine)palladium (0.65 g, 0.56 mmol), and Na₂CO₃ (7.41 g, 69.9 mmol) using Reaction Scheme d, a Boc group was removed using 4.0 M HCl (13.9 ml, 55.6 mmol in dioxane) to synthesize Compound 17, (4'-(trifluoromethoxy)-[1,1'-biphenyl]-4-yl)methanamine hydrochloride (2.73 g, 65%) in the form of a white powder.
R_{f} = 0.00 (EtOAc 9 : acetone 1);
¹H NMR (DMSO-*d₆*, 400 MHz) 8.33 (s, NH₃), 7.81-7.83 (m, ArH), 7.75 (d, *J* = 8.2 Hz, ArH), 7.59 (d, *J* = 8.2 Hz, ArH), 7.48 (d, *J* = 8.3 Hz, ArH), 4.08 (s, NH₃CH₂).

### Preparation Example 13: Preparation of 2-(3',4'-dichloro-[1,1'-biphenyl]-4-yl)ethan-1-amine hydrochloride (18)

After a compound was obtained by reacting Compound 12 (tert-butyl (4-bromophenethyl)carbamate, 1.00 g, 3.33 mmol), 3,4-dichlorophenylboronic acid (0.76 g, 4.00 mmol), tetrakis(triphenylphosphine)palladium (0.15 g, 0.15 mmol), and Na₂CO₃ (1.77 g, 16.7 mmol) using Reaction Scheme d, a Boc group was removed using 4.0 M HCl (2.50 ml, 10.0 mmol in dioxane) to synthesize Compound 18, 2-(3',4'-dichloro-[1,1'-biphenyl]-4-yl)ethan-1-amine hydrochloride (2.73 g, 65%) in the form of a white powder.
R_{f} = 0.00 (EtOAc 9 : acetone 1);
¹H NMR (DMSO-*d₆*, 400 MHz) 8.33 (s, NH₃), 7.93 (d, *J* = 1.9 Hz, ArH), 7.66-7.72 (m, ArH), 7.39 (d, *J* = 8.2 Hz, ArH), 2.98-3.07 (m, NH₃CH₂CH₂).

### Preparation Example 14: Preparation of 2-(4'-(trifluoromethyl)-[1,1'-biphenyl]-4-yl)ethan-1-amine hydrochloride (19)

After a compound was obtained by reacting Compound 12 (0.50 g, 1.67 mmol), 4-(trifluoromethyl)phenylboronic acid (0.38 g, 2.00 mmol), tetrakis(triphenylphosphine)palladium (0.08 g, 0.07 mmol), and Na₂CO₃ (0.88 g, 8.33 mmol) using Reaction Scheme d, a Boc group was removed using 4.0 M HCl (1.25 ml, 5.00 mmol in dioxane) to synthesize Compound 19, 2-(4'-(trifluoromethyl)-[1,1'-biphenyl]-4-yl)ethan-1-amine hydrochloride (0.28 g, 56%) in the form of a white powder.
R_{f} = 0.00 (EtOAc 9 : acetone 1);
¹H NMR (DMSO-*d₆*, 400 MHz) 8.37 (s, NH₃), 7.71-7.91 (m, ArH), 7.44 (d, *J* = 8.1 Hz, ArH), 3.01-3.11 (m, NH₃CH₂CH₂).

### Preparation Example 15: Preparation of 2-(4'-(trifluoromethoxy)-[1,1'-biphenyl]-4-yl)ethan-1-amine hydrochloride (20)

After a compound was obtained by reacting Compound 12 (1.50 g, 5.00 mmol), 4-(trifluoromethoxy)phenylboronic acid (1.23 g, 6.00 mmol), tetrakis(triphenylphosphine)palladium (0.23 g, 0.20 mmol), and Na₂CO₃ (2.65 g, 25.0 mmol) using Reaction Scheme d, a Boc group was removed using 4.0 M HCl (3.75 ml, 15.0 mmol in dioxane) to synthesize Compound 20, 2-(4'-(trifluoromethoxy)-[1,1'-biphenyl]-4-yl)ethan-1-amine hydrochloride (0.88 g, 55%) in the form of a white powder.
R_{f} = 0.00 (EtOAc 9 : acetone 1);
¹H NMR (DMSO-*d₆*, 400 MHz) 8.31 (s, NH₃), 7.79 (d, *J* = 8.7 Hz, ArH), 7.66 (d, *J* = 8.1 Hz, ArH), 7.45 (d, *J* = 8.2 Hz, ArH), 7.40 (d, *J* = 8.1 Hz, ArH), 2.97-3.10 (m, NH₃CH₂CH₂).

### Preparation Example 16: Preparation of 2-(3',4'-difluoro-[1,1'-biphenyl]-4-yl)ethan-1-amine hydrochloride (21)

After a compound was obtained by reacting Compound 12 (1.00 g, 3.33 mmol), 3,4-dichlorophenylboronic acid (0.76 g, 4.00 mmol), tetrakis(triphenylphosphine)palladium (0.15 g, 0.15 mmol), and Na₂CO₃ (1.77 g, 16.7 mmol) using Reaction Scheme d, a Boc group was removed using 4.0 M HCl (2.50 ml, 10.0 mmol in dioxane) to synthesize Compound 21, 2-(3',4'-difluoro-[1,1'-biphenyl]-4-yl)ethan-1-amine hydrochloride (2.73 g, 65%) in the form of a white powder.
R_{f} = 0.00 (EtOAc 9 : acetone 1);
¹H NMR (DMSO-*d₆*, 400 MHz) 7.95 (s, NH₃), 7.74-7.79 (m, ArH), 7.67 (d, *J* = 8.1 Hz, ArH), 7.48-7.54 (m, ArH), 7.37 (d, *J* = 8.1 Hz, ArH), 2.90-3.09 (m, NH₃CH₂CH₂).

### Preparation Example 17: Preparation of (R)/(S)-tert-butyl(1-((3',4'-dichloro-[1,1'-biphenyl]-4-yl)amino)-1 -oxobutan-2-yl)carbamate (22)

Compound 4 (0.63 g, 3.12 mmol), NMM (0.96 ml, 8.74 mmol), IBCF (0.53 ml, 4.06 mmol), and Compound 13 (0.90 g, 3.28 mmol) were reacted using Reaction Scheme e to synthesize Compound 22, (R)/(S)-tert-butyl(1-((3',4'-dichloro-[1,1'-biphenyl]-4-yl)amino)-1-oxobutan-2-yl)carbamate (1.09 g, 82%) in the form of a pale yellow powder.
R_{f} = 0.33 (EtOAc 1 : n-hexane 3);
¹H NMR (CDCl₃, 400 MHz) 8.55 (s, C(O)NH), 7.58 (d, *J* = 7.3 Hz, ArH), 7.44-7.47 (m, ArH), 7.34 (dd, *J* = 1.8 Hz, 8.3 Hz, ArH), 5.12 (s, Boc-NH), 4.18 (s, Chiral-H), 1.67-2.05 (m, CH₂CH₃), 1.47 (s, Boc), 1.03 (t, *J* = 7.4 Hz, CH₂CH₃).

### Preparation Example 18: Preparation of (R)/(S)-tert-butyl(1-((3',4'-dichloro-[1,1'-biphenyl]-4-yl)amino)-1-oxopentan-2-yl)carbamate (23)

Compound 5 (0.30 g, 1.52 mmol), NMM (0.42 ml, 3.80 mmol), IBCF (0.26 ml, 1.98 mmol), and Compound 13 (0.44 g, 1.60 mmol) were reacted using Reaction Scheme e to synthesize Compound 23, (R)/(S)-tert-butyl(1-((3',4'-dichloro-[1,1'-biphenyl]-4-yl)amino)-1-oxopentan-2-yl)carbamate (0.61 g, 92%) in the form of a white powder.
R_{f} = 0.37 (EtOAc 1 : n-hexane 3);
¹H NMR (CDCl₃, 400 MHz) 8.53 (s, C(O)NH), 7.62 (d, *J* = 8.6 Hz, ArH), 7.48-7.50 (m, ArH), 7.38 (dd, *J* = 2.0 Hz, 8.3 Hz, ArH), 5.08 (s, Boc-NH), 4.24 (s, Chiral-H), 1.63-1.99 (m, CH₂CH₂CH₃), 1.47-1.50 (m, Boc, CH₂CH₂CH₃), 0.99 (t, *J* = 7.3 Hz, CH₂CH₂CH₃).

### Preparation Example 19: Preparation of (R)/(S)-tert-butyl(1-((3',4'-dichloro-[1,1'-biphenyl]-4-yl)amino)-1 -oxohexan-2-yl)carbamate (24)

Compound 6 (0.80 g, 3.46 mmol), NMM (0.95 ml, 8.69 mmol), IBCF (0.58 ml, 4.50 mmol), and Compound 13 (1.00 g, 3.64 mmol) were reacted using Reaction Scheme e to synthesize Compound 24, (R)/(S)-tert-butyl(1-((3',4'-dichloro-[1,1'-biphenyl]-4-yl)amino)-1-oxohexan-2-yl)carbamate (1.11 g, 71%) in the form of a white powder.
R_{f} = 0.50 (EtOAc 1 : n-hexane 3);
¹H NMR (DMSO-*d₆*, 400 MHz) 10.10 (s, NH), 7.91 (d, *J* = 1.9 Hz, ArH), 7.64-7.74 (m, ArH), 7.04 (d, *J* = 7.8 Hz, NH), 4.02-4.07 (m, NHCHCH₂), 1.57-1.64 (m, CH₂CH₂CH₂CH₃), 1.39 (s, Boc), 1.26-1.32 (m, CH₂CH₂CH₂CH₃), 0.86 (t, *J* = 6.8 Hz, CH₂CH₂CH₂CH₃).

### Preparation Example 20: Preparation of (R)/(S)-tert-butyl(1-oxo-1-((4'-(trifluoromethoxy)-[1 ,1'-biphenyl]-4-yl)amino)pentan-2-yl)carbamate (25)

Compound 5 (0.43 g, 1.97 mmol), NMM (0.61 ml, 5.52 mmol), IBCF (0.33 ml, 2.56 mmol), and Compound 14 (0.60 g, 2.07 mmol) were reacted using Reaction Scheme e to synthesize Compound 25, (R)/(S)-tert-butyl(1-oxo-1-((4'-(trifluoromethoxy)-[1,1'-biphenyl]-4-yl)amino)pentan-2-yl)carbamate (0.66 g, 73%) in the form of a white powder.
R_{f} = 0.30 (EtOAc 1 : n-hexane 3);
¹H NMR (CDCl₃, 400 MHz) 8.62 (s, C(O)NH), 7.61 (d, *J* = 7.3 Hz, ArH), 7.54 (d, *J* = 7.6 Hz, ArH), 7.49 (d, *J* = 7.9 Hz, ArH), 7.26-7.29 (m, ArH), 5.19 (d, *J* = 7.4 Hz, Boc-NH), 4.29 (s, Chiral-H), 1.65-1.98 (m, CH₂CH₂CH₃), 1.43-1.56 (m, Boc, CH₂CH₂CH₃), 0.99 (t, *J* = 7.1 Hz, CH₂CH₂CH₃).

### Preparation Example 21: Preparation of (R)/(S)-tert-butyl(1-((3',4'-dichloro-[1,1'-biphenyl]-4-yl)amino)-1 -oxobutan-2-yl)(methyl)carbamate (26)

Compound 7 (0.68 g, 3.12 mmol), NMM (0.96 ml, 8.74 mmol), IBCF (0.53 ml, 4.06 mmol), and Compound 13 (0.90 g, 3.28 mmol) were reacted using Reaction Scheme e to synthesize Compound 26, (R)/(S)-tert-butyl(1-((3',4'-dichloro-[1,1'-biphenyl]-4-yl)amino)-1-oxobutan-2-yl)(methyl)carbamate (0.74 g, 54%) in the form of an oil.
R_{f} = 0.50 (EtOAc 1 : n-hexane 3);
¹H NMR (CDCl₃, 400 MHz) 8.50 (s, C(O)NH), 7.58-7.63 (m, ArH), 7.46-7.50 (m, ArH), 7.38 (dd, *J* = 1.8 Hz, 8.2 Hz, ArH), 4.57 (s, Chiral-H), 2.83 (s, NCH₃), 1.71-2.04 (m, CH₂CH₃), 1.51 (d, *J* = 6.8 Hz, Boc), 0.97 (t, *J* = 7.3 Hz, CH₂CH₃).

### Preparation Example 22: Preparation of (R)/(S)-tert-butyl(1-((3',4'-dichloro-[1,1'-biphenyl]-4-yl)amino)-1 -oxopentan-2-yl)(methyl)carbamate (27)

Compound 8 (0.86 g, 3.72 mmol), NMM (1.14 ml, 10.4 mmol), IBCF (0.63 ml, 4.83 mmol), and Compound 13 (1.07 g, 3.90 mmol) were reacted using Reaction Scheme e to synthesize Compound 27, (R)/(S)-tert-butyl(1-((3',4'-dichloro-[1,1'-biphenyl]-4-yl)amino)-1-oxopentan-2-yl)(methyl)carbamate (0.79 g, 47%) in the form of a yellow powder.
R_{f} = 0.48 (EtOAc 1 : n-hexane 3);
¹H NMR (CDCl₃, 400 MHz) 8.49 (s, C(O)NH), 7.58-7.64 (m, ArH), 7.47-7.51 (m, ArH), 7.38 (d, *J* = 8.3 Hz, ArH), 4.66 (s, Chiral-H), 2.82 (s, NCH₃), 1.67-2.04 (m, CH₂CH₂CH₃), 1.51 (s, Boc), 1.33-1.39 (m, CH₂CH₂CH₃), 0.99 (t, *J* = 7.3 Hz, CH₂CH₂CH₃).

### Preparation Example 23: Preparation of (R)/(S)-tert-butyl(1-((3',4'-dichloro-[1,1'-biphenyl]-4-yl)amino)-1 -oxohexan-2-yl)(methyl)carbamate (28)

Compound 9 (R)/(S)-2-((tert-butoxycarbonyl)(methyl)amino)hexanoic acid (0.84 g, 3.47 mmol), NMM (1.10 ml, 9.71 mmol), IBCF (0.58 ml, 4.51 mmol), and Compound 13 (1.00 g, 3.64 mmol) were reacted using Reaction Scheme e to synthesize Compound 28, (R)/(S)-tert-butyl(1-((3',4'-dichloro-[1,1'-biphenyl]-4-yl)amino)-1-oxohexan-2-yl)(methyl)carbamate (0.86 g, 53%) in the form of an oil.
R_{f} = 0.55 (EtOAc 1 : n-hexane 3);
¹H NMR (CDCl₃, 400 MHz) 8.49 (s, C(O)NH), 7.58-7.64 (m, ArH), 7.47-7.51 (m, ArH), 7.38 (dd, *J* = 2.1 Hz, 8.4 Hz, ArH), 4.64 (s, Chiral-H), 2.82 (s, NCH₃), 1.67-2.01 (m, CH₂CH₂CH₂CH₃), 1.52 (s, Boc), 1.24-1.44 (m, CH₂CH₂CH₂CH₃), 0.93 (t, *J* = 7.1 Hz, CH₂CH₂CH₃).

### Preparation Example 24: Preparation of (R)/(S)-2-amino-N-(3',4'-dichloro-[1,1'-biphenyl]-4-yl)butanamide hydrochloride (29)

Compound 22 (1.06 g, 2.50 mmol) and 4.0 M HCl (3.80 ml, 15.0 mmol in dioxane) were reacted using Reaction Scheme f to synthesize Compound 29, (R)/(S)-2-amino-N-(3',4'-dichloro-[1,1'-biphenyl]-4-yl)butanamide hydrochloride (0.87 g, 97%) in the form of a white powder.
R_{f} = 0.00 (EtOAc 9: acetone 1);
¹H NMR (CDCl₃, 400 MHz) 11.05 (s, C(O)NH), 8.38 (s, NH₃), 7.93 (d, *J* = 1.9 Hz, ArH), 7.66-7.79 (m, ArH), 4.01-4.04 (m, Chiral-H), 1.86-1.91 (m, CH₂CH₃), 0.96 (t, *J* = 7.5 Hz, CH₂CH₃).

### Preparation Example 25: Preparation of (R)/(S)-2-amino-N-(3',4'-dichloro-[1,1'-biphenyl]-4-yl)pentanamide hydrochloride (30)

Compound 23 (0.58 g, 1.33 mmol) and 4.0 M HCl (2.00 ml, 7.95 mmol in dioxane) were reacted using Reaction Scheme f to synthesize Compound 30, (R)/(S)-2-amino-N-(3',4'-dichloro-[1,1'-biphenyl]-4-yl)pentanamide hydrochloride (0.40 g, 81%) in the form of a white powder.
R_{f} = 0.00 (EtOAc 9: acetone 1);
¹H NMR (DMSO-*d₆*, 400 MHz) 11.05 (s, C(O)NH), 8.40 (s, NH ₃), 7.93 (d, *J* = 1.5 Hz, ArH), 7.66-7.79 (m, ArH), 4.06 (s, Chiral-H), 1.79-1.85 (m, CH₂CH₂CH₃), 1.36-1.43 (m, CH₂CH₂CH₃), 0.91 (t, *J* = 7.3 Hz, CH₂CH₂CH₃).

### Preparation Example 26: Preparation of (R)/(S)-2-amino-N-(3',4'-dichloro-[1,1'-biphenyl]-4-yl)hexanamide hydrochloride (31)

Compound 24 (1.10 g, 2.44 mmol) and 4.0 M HCl (3.66 ml, 14.6 mmol in dioxane) were reacted using Reaction Scheme f to synthesize Compound 31, (R)/(S)-2-amino-N-(3',4'-dichloro-[1,1'-biphenyl]-4-yl)hexanamide hydrochloride (0.81 g, 86%) in the form of a white powder.
R_{f} = 0.00 (EtOAc 9: acetone 1);
¹H NMR (DMSO-*d₆*, 400 MHz) 10.97 (s, C(O)NH), 8.38 (s, NH₃), 7.93 (d, *J* = 2.0 Hz, ArH), 7.66-7.78 (m, ArH), 4.03 (s, Chiral-H), 1.81-1.87 (m, CH₂CH₂CH₂CH₃), 1.33-1.39 (m, CH₂CH₂CH₂CH₃), 0.87 (t, *J* = 6.9 Hz, CH₂CH₂CH₂CH₃).

### Preparation Example 27: Preparation of (R)/(S)-2-amino-N-(4'-(trifluoromethoxy)-[1,1'-biphenyl]-4-yl)pentanamide hydrochloride (32)

Compound 25 (0.64 g, 1.41 mmol) and 4.0 M HCl (2.12 ml, 8.46 mmol in dioxane) were reacted using Reaction Scheme f to synthesize Compound 32, (R)/(S)-2-amino-N-(4'-(trifluoromethoxy)-[1 ,1'-biphenyl]-4-yl)pentanamide hydrochloride (0.51 g, 93%) in the form of a white powder.
R_{f} = 0.00 (EtOAc 9: acetone 1);
¹H NMR (DMSO-*d₆*, 400 MHz) 10.90 (s, C(O)NH), 8.35 (s, NH₃), 7.76-7.79 (m, ArH), 7.70 (d, *J* = 8.8 Hz, ArH), 7.45 (d, *J* = 8.2 Hz, ArH), 4.03 (s, Chiral-H), 1.82 (q, *J* = 6.9 Hz, 7.9 Hz, CH₂CH₂CH₃), 1.34-1.47 (m, CH₂CH₂CH₃), 0.92 (t, *J* = 7.3 Hz, CH₂CH₂CH₃).

### Preparation Example 28: Preparation of (R)/(S)-N-(3',4'-dichloro-[1,1'-biphenyl]-4-yl)-2-(methylamino)butanamide hydrochloride (33)

Compound 26 (0.69 g, 1.58 mmol) and 4.0 M HCl (2.40 ml, 9.50 mmol in dioxane) were reacted using Reaction Scheme f to synthesize Compound 33, (R)/(S)-N-(3',4'-dichloro-[1,1'-biphenyl]-4-yl)-2-(methylamino)butanamide hydrochloride (0.55 g, 93%) in the form of a white powder.
R_{f} = 0.00 (EtOAc 9: acetone 1);
¹H NMR (DMSO-*d₆*, 400 MHz) 11.00 (s, C(O)NH), 9.13 (s, NH₂), 7.94 (d, *J* = 1.7 Hz, ArH), 7.66-7.78 (m, ArH), 3.96 (t, *J* = 5.5 Hz, Chiral-H), 2.57 (s, NCH₃), 1.87-2.05 (m, CH₂CH₃), 0.94 (t, *J* = 7.5 Hz, CH₂CH₃).

### Preparation Example 29: Preparation of (R)/(S)-N-(3',4'-dichloro-[1,1'-biphenyl]-4-yl)-2-(methylamino)pentanamide hydrochloride (34)

Compound 27 (0.38 g, 0.83 mmol) and 4.0 M HCl (1.25 ml, 4.98 mmol in dioxane) were reacted using Reaction Scheme f to synthesize Compound 34, (R)/(S)-N-(3',4'-dichloro-[1,1'-biphenyl]-4-yl)-2-(methylamino)pentanamide hydrochloride (0.23 g, 71%) in the form of a white powder.
R_{f} = 0.00 (EtOAc 9: acetone 1);
¹H NMR (DMSO-*d₆*, 400 MHz) 10.98 (s, C(O)NH), 9.09 (s, NH₂), 7.94 (s, ArH), 7.66-7.76 (m, ArH), 3.96 (t, *J* = 6.1 Hz, Chiral-H), 2.57 (s, NCH₃), 1.80-1.93 (m, CH₂CH₂CH₃), 1.31-1.39 (m, CH₂CH₂CH₃), 0.91 (t, *J* = 7.3 Hz, CH₂CH₂CH₃).

### Preparation Example 30: Preparation of (R)/(S)-N-(3',4'-dichloro-[1,1'-biphenyl]-4-yl)-2-(methylamino)hexanamide hydrochloride (35)

Compound 28 (0.82 g, 1.77 mmol) and 4.0 M HCl (2.65 ml, 10.6 mmol in dioxane) were reacted using Reaction Scheme f to synthesize Compound 35, (R)/(S)-N-(3',4'-dichloro-[1,1'-biphenyl]-4-yl)-2-(methylamino)hexanamide hydrochloride (0.60 g, 84%) in the form of a white powder.
R_{f} = 0.00 (EtOAc 9: acetone 1);
¹H NMR (DMSO-*d₆*, 400 MHz) 10.81 (s, C(O)NH), 9.05 (s, NH₂), 7.94 (d, *J* = 2.0 Hz, ArH), 7.66-7.77 (m, ArH), 3.92 (s, Chiral-H), 2.57 (s, NCH ₃), 1.87-1.99 (m, CH₂CH₂CH₂CH₃), 1.29-1.33 (m, CH₂CH₂CH₂CH₃), 0.86 (t, *J* = 6.8 Hz, CH₂CH₂CH₂CH₃).

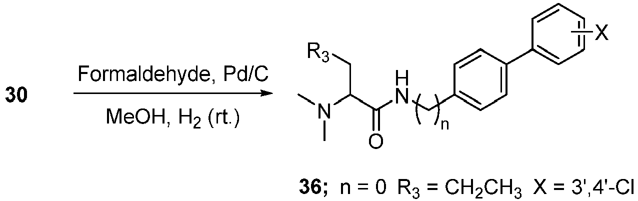

### Preparation Example 31: Preparation of (R)/(S)-N-(3',4'-dichloro-[1,1'-biphenyl]-4-yl)-2-(dimethylamino)pentanamide (36)

Compound 30 (1.0 eq), triethylamine (6.0 eq), formaldehyde (2.0 eq), and a palladium catalyst (0.4 eq) were reacted using Reaction Scheme g to synthesize Compound 36, (R)/(S)-N-(3',4'-dichloro-[1,1'-biphenyl]-4-yl)-2-(dimethylamino)pentan amide.

¹H NMR (DMSO-*d₆*, 400 MHz) 10.98 (s, C(O)NH), 7.94 (s, ArH), 7.66-7.76 (m, ArH), 3.96 (t, *J* = 6.1 Hz, Chiral-H), 2.57 (s, N(CH₃)₂), 1.80-1.93 (m, CH₂CH₂CH₃), 1.31-1.39 (m, CH₂CH₂CH₃), 0.91 (t, *J* = 7.3 Hz, CH₂CH₂CH₃).

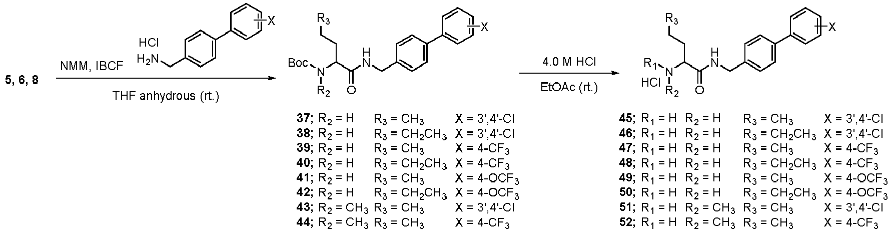

### Preparation Example 32: Preparation of (R)/(S)-tert-butyl (1-(((3',4'-dichloro-[1,1'-biphenyl]-4-yl)methyl)amino)-1-oxopentan-2-yl)carbamate (37)

Compound 5 (0.57 g, 2.64 mmol), NMM (0.73 ml, 6.60 mmol), IBCF (0.45 ml, 3.43 mmol), and Compound 15 (0.80 g, 2.77 mmol) were reacted using Reaction Scheme e to synthesize Compound 37, (R)/(S)-tert-butyl (1-((3',4'-dichloro-[1,1'-biphenyl]-4-yl)methyl)amino)-1-oxopentan-2-yl)carbamate (1.20 g, 100%) in the form of a white powder.
R_{f} = 0.04 (EtOAc 1 : n-hexane 3);
¹H NMR (CDCl₃, 400 MHz) 7.64 (d, *J* = 2.0 Hz, ArH), 7.49 (dd, *J* = 2.6 Hz, 8.5 Hz, ArH), 7.33-7.40 (m, ArH), 6.48 (s, C(O)NH), 4.93 (s, Boc-NH), 4.49 (d, *J* = 4.2 Hz, NHCH₂), 4.07-4.09 (m, Chiral-H), 1.36-1.43 (m, CH₂CH₂CH₃, Boc), 0.95 (t, *J* = 7.3 Hz, CH₂CH₂CH₃).

### Preparation Example 33: Preparation of (R)/(S)-tert-butyl (1-(((3',4'-dichloro-[1,1'-biphenyl]-4-yl)methyl)amino)-1-oxohexan-2-yl)carbamate (38)

Compound 6 (0.38 g, 1.65 mmol), NMM (0.45 ml, 4.13 mmol), IBCF (0.28 ml, 2.15 mmol), and Compound 15 (0.50 g, 1.74 mmol) were reacted using Reaction Scheme e to synthesize Compound 38, (R)/(S)-tert-butyl (1-(((3',4'-dichloro-[1,1'-biphenyl]-4-yl)methyl)amino)-1-oxohexan-2-yl)carbamate (0.46 g, 60%) in the form of a white powder.
R_{f} = 0.19 (EtOAc 1 : n-hexane 3);
¹H NMR (CDCl₃, 400 MHz) 7.63 (d, *J* = 2.0 Hz, ArH), 7.49 (dd, *J* = 4.3 Hz, 8.2 Hz, ArH), 7.33-7.39 (m, ArH), 6.55 (s, C(O)NH), 4.98 (d, *J* = 3.8 Hz, Boc-NH), 4.49 (d, *J* = 5.5 Hz, NHCH ₂), 4.07-4.11 (m, Chiral-H), 1.58-1.90 (m, CH₂CH₂CH₂CH₃), 1.42 (s, Boc), 1.34 (d, *J* = 2.2 Hz, CH₂CH₂CH₂CH₃), 0.88-0.94 (m, CH₂CH₂CH₂CH₃).

### Preparation Example 34: Preparation of (R)/(S)-tert-butyl (1-oxo-1(((4'-(trifluoromethyl)-[1,1'-biphenyl]-4-yl)methyl)amino)pentan-2-yl)carbamate (39)

Compound 5 (0.36 g, 1.66 mmol), NMM (0.46 ml, 4.14 mmol), IBCF (0.28 ml, 2.15 mmol), and Compound 16 (0.50 g, 1.74 mmol) were reacted using Reaction Scheme e to synthesize Compound 39, (R)/(S)-tert-butyl (1-oxo-1-(((4'-(trifluoromethyl)-[1,1'-biphenyl]-4-yl)methyl)amino)pentan-2-yl)carbamate (0.72 g, 96%) in the form of a white powder.
R_{f} = 0.17 (EtOAc 1 : n-hexane 3);
¹H NMR (CDCl₃, 300 MHz) 7.62-7.70 (m, ArH), 7.44 (dd, *J* = 8.1 Hz, 44.7 Hz, ArH), 6.85 (s, C(O)NH), 5.16-5.18 (m, Boc-NH), 4.47-4.49 (m, ArCH₂), 4.11-4.15 (m, Chiral-H), 1.54-1.89 (m, CH₂CH₂CH₃), 1.41 (s, Boc, CH₂CH₂CH₃), 0.93 (t, *J* = 7.2 Hz, CH₂CH₂CH₃).

### Preparation Example 35: Preparation of (R)/(S)-tert-butyl (1-oxo-1(((4'-(trifluoromethyl)-[1,1'-biphenyl]-4-yl)methyl)amino)hexan-2-yl)carbamate (40)

Compound 6 (0.54 g, 2.32 mmol), NMM (0.71 ml, 6.49 mmol), IBCF (0.39 ml, 3.01 mmol), and Compound 16 (0.70 g, 2.43 mmol) were reacted using Reaction Scheme e to synthesize Compound 40, (R)/(S)-tert-butyl (1-oxo-1-(((4'-(trifluoromethyl)-[1,1'-biphenyl]-4-yl)methyl)amino)hexan-2-yl)carbamate (0.87 g, 81%) in the form of a white powder.
R_{f} = 0.16 (EtOAc 1 : n-hexane 3);
¹H NMR (CDCl₃, 400 MHz) 7.70 (q, *J=* 4.6 Hz, 8.6 Hz, ArH), 7.57 (d, *J=* 8.2 Hz, ArH), 7.39 (d, *J* = 8.2 Hz, ArH), 6.50 (s, C(O)NH), 4.96 (s, Boc-NH), 4.53 (d, *J* = 4.7 Hz, NHCH₂), 4.09-4.10 (m, Chiral-H), 1.59-1.94 (m, CH₂CH₂CH₂CH₃), 1.45 (s, Boc), 1.37-1.38 (m, CH₂CH₂CH₂CH₃), 0.93 (t, *J=* 7.0 Hz, CH₂CH₂CH₂CH₃).

### Preparation Example 36: Preparation of (R)/(S)-tert-butyl (1-oxo-1(((4'-(trifluoromethoxy)-[1,1'-biphenyl]-4-yl)methyl)amino)pentan-2-yl)carbamate (41)

Compound 5 (0.55 g, 2.51 mmol), NMM (0.77 ml, 7.02 mmol), IBCF (0.42 ml, 3.26 mmol), and Compound 17 (0.80 g, 2.63 mmol) were reacted using Reaction Scheme e to synthesize Compound 41, (R)/(S)-tert-butyl (1-oxo-1-(((4'-(trifluoromethoxy)-[1,1'-biphenyl]-4-yl)methyl)amino)pentan-2-yl)carbamate (1.05 g, 90%) in the form of a white powder.
R_{f} = 0.09 (EtOAc 1 : n-hexane 3);
¹H NMR (CDCl₃, 400 MHz) 7.57-7.61 (m, ArH), 7.53 (d, *J* = 8.2 Hz, ArH), 7.37 (d, *J* = 8.2 Hz, ArH), 7.30 (d, *J* = 8.2 Hz, ArH), 6.47 (s, C(O)NH), 4.96 (s, Boc-NH), 4.52 (s, NHCH₂), 4.10-4.11 (m, Chiral-H), 1.59-1.94 (m, CH₂CH₂CH₃), 1.37-1.45 (m, CH₂CH₂CH₃, Boc), 0.97 (t, *J* = 7.3 Hz, CH₂CH₂CH₃).

### Preparation Example 37: Preparation of (R)/(S)-tert-butyl (1-oxo-1(((4'-(trifluoromethoxy)-[1,1'-biphenyl]-4-yl)methyl)amino)hexan-2-yl)carbamate (42)

Compound 6 (0.58 g, 2.51 mmol), NMM (0.77 ml, 7.02 mmol), IBCF (0.42 ml, 3.26 mmol), and Compound 17 (0.80 g, 2.63 mmol) were reacted using Reaction Scheme e to synthesize Compound 42, (R)/(S)-tert-butyl (1-oxo-1-(((4'-(trifluoromethoxy)-[1,1'-biphenyl]-4-yl)methyl)amino)hexan-2-yl)carbamate (1.06 g, 88%) in the form of a white powder.
R_{f} = 0.18 (EtOAc 1 : n-hexane 3);
¹H NMR (CDCl₃, 400 MHz) 7.58-7.61 (m, ArH), 7.53 (d, *J* = 8.1 Hz, ArH), 7.37 (d, *J* = 8.1 Hz, ArH), 7.30 (d, *J* = 8.5 Hz, ArH), 6.52 (s, C(O)NH), 4.99 (s, Boc-NH), 4.53 (d, *J* = 5.1 Hz, NHCH₂), 4.09-4.11 (m, Chiral-H), 1.62-1.94 (m, CH₂CH₂CH₂CH₃), 1.45 (s, Boc), 1.36-1.37 (m, CH₂CH₂CH₂CH₃), 0.92 (t, *J* = 6.9 Hz, CH₂CH₂CH₂CH₃).

### Preparation Example 38: Preparation of (R)/(S)-tert-butyl (1-(((3',4'-dichloro-[1,1'-biphenyl]-4-yl)methyl)amino)-1-oxopentan-2-yl)(methyl)carbamate (43)

Compound 8 (0.38 g, 1.65 mmol), NMM (0.46 ml, 4.13 mmol), IBCF (0.28 ml, 2.15 mmol), and Compound 15 (0.50 g, 1.73 mmol) were reacted using Reaction Scheme e to synthesize Compound 43, (R)/(S)-tert-butyl (1-(((3',4'-dichloro-[1,1'-biphenyl]-4-yl)methyl)amino)-1-oxopentan-2-yl)(methyl)carbamate (0.57 g, 73%) in the form of an oil.
R_{f} = 0.18 (EtOAc 1 : n-hexane 3);
¹H NMR (CDCl₃, 300 MHz) 7.30-7.68 (m, ArH), 6.27-6.64 (m, C(O)NH), 4.41-4.59 (m, NHCH₂, Chiral-H), 2.78 (s, NCH₃), 2.04-1.63 (m, CH₂CH₂CH₃), 1.44 (s, Boc), 1.32-1.26 (m, CH₂CH₂CH₃), 0.96 (t, *J* = 7.3 Hz, CH₂CH₂CH₃).

### Preparation Example 39: Preparation of (R)/(S)-tert-butyl methyl(1-oxo-1(((4'-(trifluoromethyl)-[1,1'-biphenyl]-4-yl)methyl)amino)pentan-2-yl)carbamate (44)

Compound 8 (0.38 g, 1.66 mmol), NMM (0.46 ml, 4.14 mmol), IBCF (0.28 ml, 2.15 mmol), and Compound 16 (0.50 g, 1.74 mmol) were reacted using Reaction Scheme e to synthesize Compound 44, (R)/(S)-tert-butyl methyl (1-oxo-1-(((4'-(trifluoromethyl)-[1,1'-biphenyl]-4-yl)methyl)amino)pentan-2-yl)carbamate (0.49 g, 64%) in the form of an oil.
R_{f} = 0.22 (EtOAc 1 : n-hexane 3);
¹H NMR (CDCl₃, 300 MHz) 7.64-7.71 (m, ArH), 7.44 (dd, *J* = 7.9 Hz, 53.4 Hz, ArH), 6.28-6.64 (m, C(O)NH), 4.43-4.61 (m, NHCH₂, Chiral-H), 2.78 (s, NCH₃), 1.63-2.04 (m, CH₂CH₂CH₃), 1.44 (s, Boc), 1.26-1.35 (m, CH₂CH₂CH₃), 0.96 (t, *J* = 7.3 Hz, CH₂CH₂CH₃).

### Preparation Example 40: Preparation of (R)/(S)-2-amino-N-((3',4'-dichloro-[1,1'-biphenyl]-4-yl)methyl)pentanamide hydrochloride (45)

Compound 37 (1.19 g, 2.64 mmol) and 4.0 M HCl (3.95 ml, 15.8 mmol in dioxane) were reacted using Reaction Scheme f to synthesize Compound 45, (R)/(S)-2-amino-N-((3',4'-dichloro-[1,1'-biphenyl]-4-yl)methyl)pentanamide hydrochloride (0.73 g, 71%) in the form of a white powder.
R_{f} = 0.00 (EtOAc 9: acetone 1);
¹H NMR (DMSO-*d₆*, 300 MHz) 9.04 (s, C(O)NH), 8.21 (s, NH ₃), 7.94 (s, ArH), 7.66-7.73 (m, ArH), 7.40 (d, *J* = 8.1 Hz, ArH), 4.39-4.41 (m, NHCH₂), 3.78-3.82 (t, *J* = 6.3 Hz, Chiral-H), 1.68-1.76 (m, CH₂CH₂CH₃), 1.29-1.39 (m, CH₂CH₂CH₃), 0.89 (t, *J* = 7.2 Hz, CH₂CH₂CH₃).

### Preparation Example 41: Preparation of (R)/(S)-2-amino-N-((3',4'-dichloro-[1,1'-biphenyl]-4-yl)methyl)hexanamide hydrochloride (46)

Compound 38 (0.46 g, 0.99 mmol) and 4.0 M HCl (1.48 ml, 1.48 mmol in dioxane) were reacted using Reaction Scheme f to synthesize Compound 46, (R)/(S)-2-amino-N-((3',4'-dichloro-[1,1'-biphenyl]-4-yl)methyl)hexanamide hydrochloride (0.27 g, 85%) in the form of a white powder.
R_{f} = 0.00 (EtOAc 9: acetone 1);
¹H NMR (DMSO-*d₆*, 400 MHz) 9.17 (m, C(O)NH), 8.32 (s, NH₃), 7.94 (d, *J* = 2.0 Hz, ArH), 7.66-7.73 (m, ArH), 7.41 (d, *J* = 8.2 Hz, ArH), 4.34-4.45 (m, NHCH₂), 3.81 (t, *J* = 6.1 Hz, Chiral-H), 1.75-1.76 (m, CH₂CH₂CH₂CH₃), 1.27-1.28 (m, CH₂CH₂CH₂CH₃), 0.85 (t, *J* = 6.6 Hz, CH₂CH₂CH₂CH₃).

### Preparation Example 42: Preparation of (R)/(S)-2-amino-N-((4'-(trifluoromethyl)-[1,1'-biphenyl]-4-yl)methyl)pentanamide hydrochloride (47)

Compound 39 (0.70 g, 1.55 mmol) and 4.0 M HCl (2.33 ml, 9.32 mmol in dioxane) were reacted using Reaction Scheme f to synthesize Compound 47, (R)/(S)-2-amino-N-(4'-(trifluoromethyl)-[1,1'-biphenyl]-4-yl)methyl)pentanamide hydrochloride (0.60 g, 99%) in the form of a white powder.
R_{f} = 0.00 (EtOAc 9: acetone 1);
¹H NMR (DMSO-*d₆*, 300 MHz) 9.37 (t, *J* = 5.7 Hz, C(O)NH), 8.46 (s, NH ₃), 7.71-7.92 (m, ArH), 7.45-7.51 (m, ArH), 4.40-4.43 (m, NHCH₂), 3.89 (s, Chiral-H), 1.74-1.82 (m, CH₂CH₂CH₃), 1.16-1.42 (m, CH₂CH₂CH₃), 0.89 (t, *J* = 7.2 Hz, CH₂CH₂CH₃).

### Preparation Example 43: Preparation of (R)/(S)-2-amino-N-((4'-(trifluoromethyl)-[1,1'-biphenyl]-4-yl)methyl)hexanamide hydrochloride (48)

Compound 40 (0.85 g, 1.83 mmol) and 4.0 M HCl (2.75 ml, 11.0 mmol in dioxane) were reacted using Reaction Scheme f to synthesize Compound 48, (R)/(S)-2-amino-N-(4'-(trifluoromethyl)-[1,1'-biphenyl]-4-yl)methyl)hexanamide hydrochloride (0.72 g, 98%) in the form of a white powder.
R_{f} = 0.00 (EtOAc 9: acetone 1);
¹H NMR (DMSO-*d₆*, 400 MHz) 9.09 (s, C(O)NH), 8.23 (s, NH₃), 7.89 (d, *J* = 8.2 Hz, ArH), 7.82 (d, *J* = 8.4 Hz, ArH), 7.73 (d, *J* = 8.1 Hz, ArH), 7.44 (d, *J* = 8.1 Hz, ArH), 4.36-4.46 (m, NHCH₂), 3.80 (t, *J* = 6.4 Hz, Chiral-H), 1.74-1.76 (m, CH₂CH₂CH₂CH₃), 1.27-1.29 (m, CH₂CH₂CH₂CH₃), 0.85 (t, *J* = 6.5 Hz, CH₂CH₂CH₂CH₃).

### Preparation Example 44: Preparation of (R)/(S)-2-amino-N-((4'-(trifluoromethoxy)-[1,1'-biphenyl]-4-yl)methyl)pentanamide hydrochloride (49)

Compound 41 (1.04 g, 2.22 mmol) and 4.0 M HCl (3.34 ml, 13.3 mmol in dioxane) were reacted using Reaction Scheme f to synthesize Compound 49, (R)/(S)-2-amino-N-((4'-(trifluoromethoxy)-[1 ,1'-biphenyl]-4-yl)methyl)pentanamide hydrochloride (0.82 g, 92%) in the form of a white powder.
R_{f} = 0.00 (EtOAc 9: acetone 1);
¹H NMR (DMSO-*d₆*, 400 MHz) 9.14 (t, *J* = 5.7 Hz, C(O)NH), 8.30 (s, NH₃), 7.77-7.81 (m, ArH), 7.67 (d, *J* = 8.2 Hz, ArH), 7.46 (d, *J* = 8.1 Hz, ArH), 7.41 (d, *J* = 8.2 Hz, ArH), 4.40 (d, *J* = 5.8 Hz, NHCH₂), 3.82 (t, *J* = 6.5 Hz, Chiral-H), 1.71-1.76 (m, CH₂CH₂CH₃), 1.28-1.38 (m, CH₂CH₂CH₃), 0.89 (t, *J* = 7.3 Hz, CH₂CH₂CH₃).

### Preparation Example 45: Preparation of (R)/(S)-2-amino-N-((4'-(trifluoromethoxy)-[1,1'-biphenyl]-4-yl)methyl)hexanamide hydrochloride (50)

Compound 42 (1.04 g, 2.17 mmol) and 4.0 M HCl (3.26 ml, 13.0 mmol in dioxane) were reacted using Reaction Scheme f to synthesize Compound 50, (R)/(S)-2-amino-N-((4'-(trifluoromethoxy)-[1 ,1'-biphenyl]-4-yl)methyl)hexanamide hydrochloride (0.88 g, 97%) in the form of a white powder.
R_{f} = 0.00 (EtOAc 9: acetone 1);
¹H NMR (DMSO-*d₆*, 400 MHz) 9.09 (t, J = 5.8 Hz, C(O)NH), 8.25 (s, NH ₃), 7.78 (d, J = 8.7 Hz, ArH), 7.66 (d, *J* = 8.2 Hz, ArH), 7.45 (d, *J* = 8.3 Hz, ArH), 7.41 (d, *J* = 8.2 Hz, ArH), 4.35-4.44 (m, NHCH₂), 3.80 (t, *J* = 6.4 Hz, Chiral-H), 1.72-1.75 (m, CH₂CH₂CH₂CH₃), 1.27-1.28 (m, CH₂CH₂CH₂CH₃), 0.85 (t, *J* = 6.5 Hz, CH₂CH₂CH₂CH₃).

### Preparation Example 46: Preparation of (R)/(S)-N-((3',4'-dichloro-[1,1'-biphenyl]-4-yl)methyl)-2-(methylamino)pentanamide hydrochloride (51)

Compound 43 (0.84 g, 1.81 mmol) and 4.0 M HCl (2.80 ml, 10.9 mmol in dioxane) were reacted using Reaction Scheme f to synthesize Compound 51, (R)/(S)-N-(3',4'-dichloro-[1,1'-biphenyl]-4-yl)methyl)-2-(methylamino)pentanamide hydrochloride (0.64 g, 88%) in the form of a white powder.
R_{f} = 0.00 (EtOAc 9: acetone 1);
¹H NMR (DMSO-*d₆*, 300 MHz) 9.10-9.78 (m, NH₂), 9.57 (t, *J* = 5.5 Hz, C(O)NH), 7.96 (s, ArH), 7.69-7.72 (m, ArH), 7.43 (d, *J* = 10.7 Hz, ArH), 4.42-4.44 (m, NHCH₂), 3.85-3.90 (m, Chiral-H), 2.48 (s, NCH₃), 1.76-1.90 (m, CH₂CH₂CH₃), 1.26-1.38 (m, CH₂CH₂CH₃), 0.90 (t, *J* = 7.1 Hz, CH₂CH₂CH₃).

### Preparation Example 47: Preparation of (R)/(S)-2-(methylamino)-N-((4'-(trifluoromethyl)-[1,1'-biphenyl]-4-yl)methyl)pentanamide hydrochloride (52)

Compound 44 (0.45 g, 0.97 mmol) and 4.0 M HCl (1.45 ml, 5.81 mmol in dioxane) were reacted using Reaction Scheme f to synthesize Compound 52, (R)/(S)-2-(methylamino)-N-((4'-(trifluoromethyl)-[1,1'-biphenyl]-4-yl)methyl)pentanamide hydrochloride (0.32 g, 82%) in the form of a white powder.
R_{f} = 0.00 (EtOAc 9: acetone 1);
¹H NMR (DMSO-*d₆*, 300 MHz) 9.03-9.81 (m, NH ₂), 9.52 (t, *J* = 5.5 Hz, C(O)NH), 7.72-7.92 (m, ArH), 7.46 (d, *J* = 8.2 Hz, ArH), 4.43-4.45 (m, NHCH ₂), 3.86 (s, Chiral-H), 2.48 (s, NCH₃), 1.77-1.89 (m, CH₂CH₂CH₃), 1.25-1.38 (m, CH₂CH₂CH₃), 0.90 (t, *J* = 7.2 Hz, CH₂CH₂CH₃).

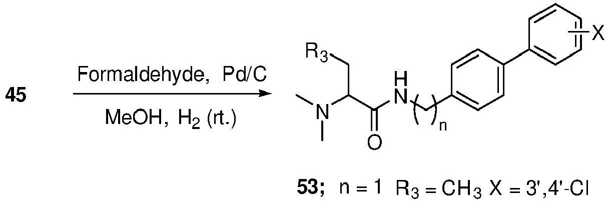

### Preparation Example 48: Preparation of (R)/(S)-N-((3',4'-dichloro-[1,1'-biphenyl]-4-yl)methyl)-2-(dimethylamino)pentanamide (53)

Compound 45 (1.0 eq), triethylamine (6.0 eq), formaldehyde (1.05 eq), and a palladium catalyst (0.2 eq) were reacted using Reaction Scheme g to synthesize Compound 53, (R)/(S)-N-((3',4'-dichloro-[1,1'-biphenyl]-4-yl)methyl)-2-(dimethylamino)pentanamide.

¹H NMR (DMSO-*d₆*, 300 MHz) 9.57 (t, *J* = 5.5 Hz, C(O)NH), 7.96 (s, ArH), 7.69-7.72 (m, ArH), 7.43 (d, *J* = 10.7 Hz, ArH), 4.42-4.44 (m, NHCH₂), 3.85-3.90 (m, Chiral-H), 2.48 (s, N(CH₃)₂), 1.76-1.90 (m, CH₂CH₂CH₃), 1.26-1.38 (m, CH₂CH₂CH₃), 0.90 (t, *J* = 7.1 Hz, CH₂CH₂CH₃).

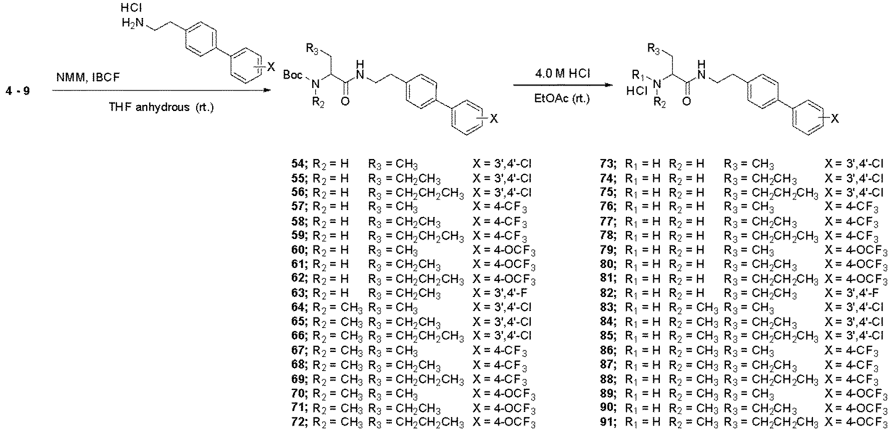

### Preparation Example 49: Preparation of (R)/(S)-tert-butyl (1-((2-(3',4'-dichloro-[1,1'-biphenyl]-4-yl)ethyl)amino)-1-oxobutan-2-yl)carbamate (54)

Compound 4 (0.32 g, 1.57 mmol), NMM (0.43 ml, 3.93 mmol), IBCF (0.27 ml, 2.05 mmol), and Compound 18 (0.50 g, 1.65 mmol) were reacted using Reaction Scheme e to synthesize Compound 54, (R)/(S)-tert-butyl (1-((2-(3',4'-dichloro-[1,1'-biphenyl]-4-yl)ethyl)amino)-1-oxobutan-2-yl)carbamate (0.66 g, 93%) in the form of a white powder.
R_{f} = 0.05 (EtOAc 1 : n-hexane 3);
¹H NMR (CDCl₃, 400 MHz) 7.65 (s, ArH), 7.47-7.50 (m, ArH), 7.39 (d, *J* = 8.2 Hz, ArH), 7.26-7.28 (m, ArH), 6.07 (s, C(O)NH), 4.94 (s, Boc-NH), 3.93 (d, *J* = 6.6 Hz, Chiral-H), 3.50-3.94 (m, NHCH₂CH₂), 2.86 (t, *J* = 6.9 Hz, NHCH₂CH₂), 1.55-2.88 (m, CH₂CH₃), 1.43 (s, Boc), 0.91 (t, *J* = 7.4 Hz, CH₂CH₃).

### Preparation Example 50: Preparation of (R)/(S)-tert-butyl (1-((2-(3',4'-dichloro-[1,1'-biphenyl]-4-yl)ethyl)amino)-1-oxopentan-2-yl)carbamate (55)

Compound 5 (0.50 g, 2.30 mmol), NMM (0.51 ml, 4.60 mmol), IBCF (0.39 ml, 2.99 mmol), and Compound 18 (0.73 g, 2.42 mmol) were reacted using Reaction Scheme e to synthesize Compound 55, (R)/(S)-tert-butyl (1-((2-(3',4'-dichloro-[1,1'-biphenyl]-4-yl)ethyl)amino)-1-oxopentan-2-yl)carbamate (0.96 g, 89%) in the form of a white powder.
R_{f} = 0.26 (EtOAc 1 : n-hexane 3);
¹H NMR (CDCl₃, 400 MHz) 7.46-7.64 (m, ArH), 7.26-7.40 (m, ArH), 6.36 (s, C(O)NH), 5.07 (s, Boc-NH), 4.01-4.03 (m, Chiral-H), 3.47-3.61 (m, NHCH₂CH₂), 2.85 (t, *J* = 7.0 Hz, NHCH₂CH₂), 2.67 (s, NCH₃), 1.48-1.80 (m, CH₂CH₂CH₃), 1.42 (s, Boc), 1.26-1.36 (m, CH₂CH₂CH₃), 0.90 (t, *J* = 7.2 Hz, CH₂CH₂CH₃).

### Preparation Example 51: Preparation of (R)/(S)-tert-butyl (1-((2-(3',4'-dichloro-[1,1'-biphenyl]-4-yl)ethyl)amino)-1-oxohexan-2-yl)carbamate (56)

Compound 6 (0.36 g, 1.57 mmol), NMM (0.43 ml, 3.93 mmol), IBCF (0.27 g, 2.05 mmol), and Compound 18 (0.50 g, 16.5 mmol) were reacted using Reaction Scheme e to synthesize Compound 56, (R)/(S)-tert-butyl (1-((2-(3',4'-dichloro-[1,1'-biphenyl]-4-yl)ethyl)amino)-1-oxohexan-2-yl)carbamate (0.76 g, 100%) in the form of a white powder.
R_{f} = 0.07 (EtOAc 1 : n-hexane 3);
¹H NMR (CDCl₃, 400 MHz) 7.65 (s, ArH), 7.48 (d, *J* = 6.3 Hz, ArH), 7.39 (d, *J* = 8.0 Hz, ArH), 7.26-7.28 (m, ArH), 6.07 (s, C(O)NH), 4.91 (s, Boc-NH), 3.97 (d, *J* = 5.6 Hz, Chiral-H), 3.51-3.62 (m, NHCH₂CH₂), 2.86 (t, *J* = 6.1 Hz, NHCH₂CH₂), 1.51-1.81 (m, CH₂CH₂CH₂CH₃), 1.42 (s, Boc), 1.27 (d, *J* = 6.7 Hz, CH₂CH₂CH₂CH₃), 0.87 (d, *J* = 5.5 Hz, CH₂CH₂CH₂CH₃).

### Preparation Example 52: Preparation of (R)/(S)-tert-butyl (1-oxo-1-((2-(4'-(trifluoromethyl)-[1,1'-biphenyl]-4-yl)ethyl)amino)butan-2-yl)carbamate (57)

Compound 4 (0.42 g, 2.08 mmol), NMM (0.57 ml, 5.21 mmol), IBCF (0.35 ml, 2.71 mmol), and Compound 19 (0.66 g, 2.19 mmol) were reacted using Reaction Scheme e to synthesize Compound 57, (R)/(S)-tert-butyl (1-oxo-1-((2-(4'-(trifluoromethyl)-[1,1'-biphenyl]-4-yl)ethyl)amino)butan-2-yl)carbamate (0.92 g, 98%) in the form of a white powder.
R_{f} = 0.16 (EtOAc 1 : n-hexane 3);
¹H NMR (CDCl₃, 400 MHz) 7.70 (s, ArH), 7.57 (d, *J* = 8.0 Hz, ArH), 7.29-7.33 (m, ArH), 6.15 (s, C(O)NH), 5.00 (s, Boc-NH), 3.94-3.99 (m, Chiral-H), 3.53-3.66 (m, NHCH₂CH₂), 2.90 (t, *J* = 7.0 Hz, NHCH₂CH₂), 1.58-1.90 (m, CH₂CH₃), 1.45 (s, Boc), 0.93 (t, *J* = 7.5 Hz, CH₂CH₃).

### Preparation Example 53: Preparation of (R)/(S)-tert-butyl (1-oxo-1-((2-(4'-(trifluoromethyl)-[1,1'-biphenyl]-4-yl)ethyl)amino)pentan-2-yl)carbamate (58)

Compound 5 (0.55 g, 2.53 mmol), NMM (0.69 ml, 6.31 mmol), IBCF (0.43 ml, 3.28 mmol), and Compound 19 (0.80 g, 2.65 mmol) were reacted using Reaction Scheme e to synthesize Compound 58, (R)/(S)-tert-butyl (1-oxo-1-((2-(4'-(trifluoromethyl)-[1,1'-biphenyl]-4-yl)ethyl)amino)pentan-2-yl)carbamate (1.05 g, 89%) in the form of a white powder.
R_{f} = 0.20 (EtOAc 1 : n-hexane 3);
¹H NMR (CDCl₃, 300 MHz) 7.67 (s, ArH), 7.41 (dd, *J* = 8.1 Hz, 63.6 Hz, ArH), 6.36 (s, C(O)NH), 5.05-5.07 (m, Boc-NH), 4.00-4.05 (m, Chiral-H), 3.45-3.66 (m, NHCH₂CH₂), 2.87 (t, *J* = 7.1 Hz, NHCH₂CH₂), 1.50-1.83 (m, CH₂CH₂CH₃), 1.42 (s, Boc), 1.28-1.39 (m, CH₂CH₂CH₃), 0.90 (t, *J* = 7.2 Hz, CH₂CH₂CH₃).

### Preparation Example 54: Preparation of (R)/(S)-tert-butyl (1-oxo-1-((2-(4'-(trifluoromethyl)-[1,1'-biphenyl]-4-yl)ethyl)amino)carbamate (59)

Compound 6 (0.76 g, 3.16 mmol), NMM (0.87 ml, 7.89 mmol), IBCF (0.53 ml, 4.10 mmol), and Compound 19 (1.00 g, 3.31 mmol) were reacted using Reaction Scheme e to synthesize Compound 59, (R)/(S)-tert-butyl (1-oxo-1-((2-(4'-(trifluoromethyl)-[1,1'-biphenyl]-4-yl)ethyl)amino)carbamate (1.34 g, 89%) in the form of a white powder.
R_{f} = 0.13 (EtOAc 1 : n-hexane 3);
¹H NMR (DMSO-*d₆*, 400 MHz) 7.87 (d, *J* = 8.2 Hz, ArH, C(O)NH), 7.80 (d, *J* = 8.5 Hz, ArH), 7.66 (d, *J* = 8.1 Hz, ArH), 7.35 (d, *J* = 8.1 Hz, ArH), 6.73 (d, *J* = 8.2 Hz, Boc-NH), 3.83 (q, *J* = 5.6 Hz, 8.4 Hz, Chiral-H), 3.24-3.43 (m, NHCH₂CH₂), 2.77 (t, *J* = 7.0 Hz, NHCH₂CH₂), 1.37-1.52 (m, CH₂CH₂CH₂CH₃, Boc), 1.15-1.20 (m, CH₂CH₂CH₂CH₃), 0.80 (t, *J* = 6.8 Hz, CH₂CH₂CH₂CH₃).

### Preparation Example 55: Preparation of (R)/(S)-tert-butyl (1-oxo-1-((2-(4'-(trifluoromethoxy)-[1,1'-biphenyl]-4-yl)ethyl)amino)butan-2-yl)carbamate (60)

Compound 4 (0.49 g, 2.40 mmol), NMM (0.66 ml, 6.00 mmol), IBCF (0.41 ml, 3.12 mmol), and Compound 20 (0.80 g, 2.52 mmol) were reacted using Reaction Scheme e to synthesize Compound 60, (R)/(S)-tert-butyl (1-oxo-1-((2-(4'-(trifluoromethoxy)-[1,1'-biphenyl]-4-yl)ethyl)amino)butan-2-yl)carbamate (0.94 g, 84%) in the form of a white powder.
R_{f} = 0.14 (EtOAc 1 : n-hexane 3);
¹H NMR (CDCl₃, 400 MHz) 7.68-7.73 (m, ArH), 7.51-7.62 (m, ArH), 7.29-7.33 (m, ArH), 6.17 (d, *J* = 5.6 Hz, C(O)NH), 5.01 (s, NH), 3.95-3.98 (m, Chiral-H), 3.51-3.67 (m, NHCH₂CH₂), 2.87-2.92 (m, NHCH₂CH₂), 1.56-1.92 (m, CH₂CH₃), 1.45 (s, Boc), 0.93 (t, *J* = 7.4 Hz, CH₂CH₃).

### Preparation Example 56: Preparation of (R)/(S)-tert-butyl (1-oxo-1-((2-(4'-(trifluoromethoxy)-[1,1'-biphenyl]-4-yl)ethyl)amino)pentan-2-yl)carbamate (61)

Compound 5 (0.33 g, 1.50 mmol), NMM (0.41 ml, 3.75 mmol), IBCF (0.25 ml, 1.95 mmol), and Compound 20 (0.50 g, 1.57 mmol) were reacted using Reaction Scheme e to synthesize Compound 61, (R)/(S)-tert-butyl (1-oxo-1-((2-(4'-(trifluoromethoxy)-[1,1'-biphenyl]-4-yl)ethyl)amino)pentan-2-yl)carbamate (0.70 g, 98%) in the form of a white powder.
R_{f} = 0.12 (EtOAc 1 : n-hexane 3);
¹H NMR (DMSO-*d₆*, 400 MHz) 7.88 (t, *J* = 5.6 Hz, C(O)NH), 7.74-7.77 (m, ArH), 7.59 (d, *J* = 8.2 Hz, ArH), 7.44 (d, *J* = 8.1 Hz, ArH), 7.32 (d, *J* = 8.2 Hz, ArH), 6.74 (d, *J* = 8.2 Hz, Boc-NH), 4.86 (q, *J* = 5.6 Hz, 8.2 Hz, Chiral-H), 3.26-3.40 (m, NHCH₂CH₂), 2.76 (t, *J* = 7.0 Hz, NHCH₂CH₂), 1.37-1.52 (m, CH₂CH₂CH₃, Boc), 1.17-1.25 (m, CH₂CH₂CH₃), 0.81 (t, J = 7.3 Hz, CH₂CH₂CH₃).

### Preparation Example 57: Preparation of (R)/(S)-tert-butyl (1-oxo-1-((2-(4'-(trifluoromethoxy)-[1,1'-biphenyl]-4-yl)ethyl)amino)hexan-2-yl)carbamate (62)

Compound 6 (0.50 g, 2.18 mmol), NMM (0.60 ml, 5.45 mmol), IBCF (0.37 ml, 2.83 mmol), and Compound 20 (0.72 g, 2.29 mmol) were reacted using Reaction Scheme e to synthesize Compound 62, (R)/(S)-tert-butyl (1-oxo-1-((2-(4'-(trifluoromethoxy)-[1,1'-biphenyl]-4-yl)ethyl)amino)hexan-2-yl)carbamate (0.95 g, 88%) in the form of a white powder.
R_{f} = 0.13 (EtOAc 1 : n-hexane 3);
¹H NMR (CDCl₃, 400 MHz) 7.70-7.73 (m, ArH), 7.57 (d, *J* = 8.1 Hz, ArH), 7.32 (d, *J* = 8.1 Hz, ArH), 6.15 (s, C(O)NH), 4.95 (s, Boc-NH), 4.00 (q, *J* = 6.4 Hz, 7.2 Hz, Chiral-H), 3.53-3.65 (m, NHCH₂CH₂), 2.90 (t, *J* = 7.0 Hz, NHCH₂CH₂), 1.52-1.85 (m, CH₂CH₂CH₂CH₃), 1.45 (s, Boc), 1.31-1.34 (m, CH₂CH₂CH₂CH₃), 0.90 (t, *J* = 6.9 Hz, CH₂CH₂CH₂CH₃).

### Preparation Example 58: Preparation of (R)/(S)-tert-butyl (1-((2-(3',4'-difluoro-[1,1'-biphenyl]-4-yl)ethyl)amino)-1-oxopentan-2-yl)carbamate (63)

Compound 5 (0.50 g, 2.29 mmol), NMM (0.70 ml, 6.40 mmol), IBCF (0.39 ml, 2.97 mmol), and Compound 21 (0.80 g, 2.40 mmol) were reacted using Reaction Scheme e to synthesize Compound 63, (R)/(S)-tert-butyl (1-((2-(3',4'-difluoro-[1,1'-biphenyl]-4-yl)ethyl)amino)-1-oxopentan-2-yl)carbamate (0.97 g, 98%) in the form of a white powder.
R_{f} = 0.10 (EtOAc 1 : n-hexane 3);
¹H NMR (CDCl₃, 400 MHz) 7.46 (d, *J* = 7.9 Hz, ArH), 7.33-7.38 (m, ArH), 7.17-7.27 (m, ArH), 6.11 (s, C(O)NH), 4.92 (s, Boc-NH), 3.96-4.01 (m, Chiral-H), 3.47-3.63 (m, NHCH₂CH₂), 2.85 (t, *J* = 7.0 Hz, NHCH₂CH₂), 1.48-1.82 (m, CH₂CH₂CH₃), 1.42 (s, Boc), 1.26-1.36 (m, CH₂CH₂CH₃), 0.90 (t, *J* = 7.3 Hz, CH₂CH₂CH₃).

### Preparation Example 59: Preparation of (R)/(S)-tert-butyl (1-((2-(3',4'-dichloro-[1,1'-biphenyl]-4-yl)ethyl)amino)-1-oxobutan-2-yl)(methyl)carbamate (64)

Compound 7 (0.82 g, 3.78 mmol), NMM (1.04 ml, 9.44 mmol), IBCF (0.64 ml, 4.91 mmol), and Compound 18 (1.20 g, 3.97 mmol) were reacted using Reaction Scheme e to synthesize Compound 64, (R)/(S)-tert-butyl (1-((2-((3',4'-dichloro-[1,1'-biphenyl]-4-yl)ethyl)amino)-1-oxobutan-2-yl)(methyl)carbamate (1.38 g, 79%) in the form of an oil.
R_{f} = 0.22 (EtOAc 1 : n-hexane 3);
¹H NMR (CDCl₃, 400 MHz) 7.66 (d, *J* = 2.0 Hz, ArH), 7.49-7.53 (m, ArH), 7.41 (dd, *J* = 2.1 Hz, 6.3 Hz, ArH), 7.27-7.29 (m ArH), 6.26 (s, C(O)NH), 4.44 (s, Chiral-H), 3.55-3.64 (m, NHCH₂CH₂), 2.87 (t, *J* = 6.7 Hz, NHCH₂CH₂), 2.70 (s, NCH ₃), 1.61-1.98 (m, CH₂CH₃), 1.45 (s, Boc), 0.88 (t, *J* = 7.4 Hz, CH₂CH₃).

### Preparation Example 60: Preparation of (R)/(S)-tert-butyl (1-((2-(3',4'-dichloro-[1,1'-biphenyl]-4-yl)ethyl)amino)-1-oxopentan-2-yl)(methyl)carbamate (65)

Compound 8 (0.35 g, 1.51 mmol), NMM (0.33 ml, 3.03 mmol), IBCF (0.26 ml, 1.97 mmol), and Compound 18 (0.48 g, 1.59 mmol) were reacted using Reaction Scheme e to synthesize Compound 65, (R)/(S)-tert-butyl (1-((2-(3',4'-dichloro-[1,1'-biphenyl]-4-yl)ethyl)amino)-1-oxopentan-2-yl)(methyl)carbamate (0.69 g, 95%) in the form of an oil.
R_{f} = 0.33 (EtOAc 1 : n-hexane 3);
¹H NMR (CDCl₃, 400 MHz) 7.46-7.64 (m, ArH), 7.35-7.40 (m, ArH), 7.25-7.27 (m, ArH), 5.95-6.25 (m, C(O)NH), 4.51 (s, Chiral-H), 3.53-3.60 (m, NHCH₂CH₂), 2.84 (t, *J* = 6.6 Hz, NHCH₂CH₂), 2.67 (s, NCH₃), 1.57-1.90 (m, CH₂CH₂CH₃), 1.42 (s, Boc), 1.22-1.32 (m, CH₂CH₂CH₃), 0.95 (t, *J* = 7.3 Hz, CH₂CH₂CH₃).

### Preparation Example 61: Preparation of (R)/(S)-tert-butyl (1-((2-(3',4'-dichloro-[1,1'-biphenyl]-4-yl)ethyl)amino)-1-oxohexan-2-yl)(methyl)carbamate (66)

Compound 9 (0.29 g, 1.20 mmol), NMM (0.33 ml, 2.99 mmol), IBCF (0.20 ml, 0.16 mmol), and Compound 18 (0.38 g, 1.26 mmol) were reacted using Reaction Scheme e to synthesize Compound 66, (R)/(S)-tert-butyl (1-((2-((3',4'-dichloro-[1,1'-biphenyl]-4-yl)ethyl)amino)-1-oxohexan-2-yl)(methyl)carbamate (0.60 g, 97%) in the form of an oil.
R_{f} = 0.30 (EtOAc 1 : n-hexane 3);
¹H NMR (DMSO-*d₆*, 400 MHz) 7.89 (d, *J* = 1.9 Hz, ArH, C(O)NH), 7.62-7.71 (m, ArH), 7.30 (d, *J* = 8.1 Hz, ArH), 4.27-4.47 (m, Chiral-H), 3.32-3.35 (m, NHCH₂CH₂), 2.77 (t, *J* = 6.5 Hz, NHCH₂CH₂), 2.67 (s, NCH₃), 1.53-1.71 (m, CH₂CH₂CH₂CH₃), 1.39 (s, Boc), 1.12-1.28 (m, CH₂CH₂CH₂CH₃), 0.84 (s, CH₂CH₂CH₂CH₃).

### Preparation Example 62: Preparation of (R)/(S)-tert-butyl methyl(1-oxo-1((2-(4'-(trifluoromethyl)-[1,1'-biphenyl]-4-yl)ethyl)amino)butan-2-yl)carbamate (67)

Compound 7 (0.62 g, 2.84 mmol), NMM (0.87 ml, 7.95 mmol), IBCF (0.48 ml, 3.69 mmol), and Compound 19 (0.90 g, 2.98 mmol) were reacted using Reaction Scheme e to synthesize Compound 67, (R)/(S)-tert-butyl methyl(1-oxo-1-((2-(4'-(trifluoromethyl)-[1,1'-biphenyl]-4-yl)ethyl)amino)butan-2-yl)carbamate (0.89 g, 67%) in the form of a white powder.
R_{f} = 0.15 (EtOAc 1 : n-hexane 3);
¹H NMR (CDCl₃, 400 MHz) 7.70 (t, *J* = 9.7 Hz, ArH), 7.56 (d, *J* = 7.9 Hz, ArH), 7.28-7.32 (m, ArH), 5.94-6.22 (m, C(O)NH), 4.45 (s, Chiral-H), 3.57-3.64 (m, NHCH₂CH₂), 2.89 (d, *J=* 6.1 Hz, NHCH₂CH₂), 2.70 (s, NCH₃), 1.61-1.99 (m, CH₂CH₃), 1.45 (s, Boc), 0.89 (t, *J=* 7.3 Hz, CH₂CH₃).

### Preparation Example 63: Preparation of (R)/(S)-tert-butyl methyl(1-oxo-1((2-(4'-(trifluoromethyl)-[1,1'-biphenyl]-4-yl)ethyl)amino)pentan-2-yl)carbamate (68)

Compound 8 (0.58 g, 2.53 mmol), NMM (0.69 ml, 6.31 mmol), IBCF (0.43 ml, 3.28 mmol), and Compound 19 (0.80 g, 2.65 mmol) were reacted using Reaction Scheme e to synthesize Compound 68, (R)/(S)-tert-butyl methyl (1-oxo-1-((2-(4'-(trifluoromethyl)-[1,1'-biphenyl]-4-yl)ethyl)amino)pentan-2-yl)carbamate (1.19 g, 98%) in the form of an oil.
R_{f} = 0.26 (EtOAc 1 : n-hexane 3);
¹H NMR (CDCl₃, 300 MHz) 7.64-7.70 (m, ArH), 7.41 (dd, *J* = 8.1 Hz, 67.4 Hz, ArH), 5.95-6.21 (s, C(O)NH), 4.52 (s, Chiral-H), 3.54-3.63 (m, NHCH₂CH₂), 2.86 (t, *J* = 6.8 Hz, NHCH₂CH₂), 2.67 (s, NCH₃), 1.55-1.91 (m, CH₂CH₂CH₃), 1.42 (s, Boc), 1.23-1.27 (m, CH₂CH₂CH₃), 0.88-0.95 (m, CH₂CH₂CH₃).

### Preparation Example 64: Preparation of (R)/(S)-tert-butyl methyl((1-oxo-1((2-(4'-(trifluoromethyl)-[1,1'-biphenyl]-4-yl)ethyl)amino)hexan-2-yl)carbamate (69)

Compound 9 (0.70 g, 2.84 mmol), NMM (0.87 ml, 7.95 mmol), IBCF (0.48 ml, 3.69 mmol), and Compound 19 (0.90 g, 2.98 mmol) were reacted using Reaction Scheme e to synthesize Compound 69, (R)/(S)-tert-butyl methyl(1-oxo-1-((2-(4'-(trifluoromethyl)-[1,1'-biphenyl]-4-yl)ethyl)amino)hexan-2-yl)carbamate (0.88 g, 63%) in the form of a yellow powder.
R_{f} = 0.32 (EtOAc 1 : n-hexane 3);
¹H NMR (DMSO-*d₆*, 400 MHz) 7.87 (d, *J* = 8.3 Hz, ArH, C(O)NH), 7.80 (d, *J* = 8.4 Hz, ArH), 7.66 (d, *J* = 8.2 Hz, ArH), 7.33 (d, *J* = 8.1 Hz, ArH), 4.27-4.47 (m, Chiral-H), 3.32-3.33 (m, NHCH₂CH₂), 2.78 (t, *J* = 6.8 Hz, NHCH₂CH₂), 2.66 (s, NCH₃), 1.53-1.71 (m, CH₂CH₂CH₂CH₃), 1.39 (s, Boc), 1.12-1.28 (m, CH₂CH₂CH₂CH₃), 0.84-0.89 (m, CH₂CH₂CH₂CH₃).

### Preparation Example 65: Preparation of (R)/(S)-tert-butyl methyl(1-oxo-1((2-(4'-(trifluoromethoxy)-[1 ,1'-biphenyl]-4-yl)ethyl)amino)butan-2-yl)carbamate (70)

Compound 7 (0.65 g, 3.00 mmol), NMM (0.92 ml, 8.99 mmol), IBCF (0.51 ml, 3.90 mmol), and Compound 20 (1.00 g, 3.15 mmol) were reacted using Reaction Scheme e to synthesize Compound 70, (R)/(S)-tert-butyl methyl (1-oxo-1-((2-(4'-(trifluoromethoxy)-[1,1'-biphenyl]-4-yl)ethyl)amino)butan-2-yl)carbamate (1.00 g, 69%) in the form of an oil.
R_{f} = 0.19 (EtOAc 1 : n-hexane 3);
¹H NMR (CDCl₃, 400 MHz) 7.51-7.70 (m, ArH), 7.29 (t, *J* = 3.7 Hz, ArH), 5.98-6.26 (m, C(O)NH), 4.45 (s, Chiral-H), 3.59-3.60 (m, NHCH₂CH₂), 2.87 (s, NHCH₂CH₂), 2.70 (d, *J* = 3.2
Hz, NCH₃), 1.62-2.07 (m, CH₂CH₃), 1.45 (s, Boc), 0.90 (m, CH₂CH₃).

### Preparation Example 66: Preparation of (R)/(S)-tert-butyl methyl(1-oxo-1((2-(4'-(trifluoromethoxy)-[1,1'-biphenyl]-4-yl)ethyl)amino)pentan-2-yl)carbamate (71)

Compound 8 (0.25 g, 1.08 mmol), NMM (0.30 ml, 2.69 mmol), IBCF (0.18 ml, 1.40 mmol), and Compound 20 (0.36 g, 1.13 mmol) were reacted using Reaction Scheme e to synthesize Compound 71, (R)/(S)-tert-butyl methyl(1-oxo-1-((2-(4'-(trifluoromethoxy)-[1,1'-biphenyl]-4-yl)ethyl)amino)pentan-2-yl)carbamate (0.32 g, 60%) in the form of a white powder.
R_{f} = 0.18 (EtOAc 1 : n-hexane 3);
¹H NMR (DMSO-*d₆*, 400 MHz) 7.89 (s, C(O)NH), 7.76 (d, *J* = 8.4 Hz, ArH), 7.59 (d, *J* = 7.8 Hz, ArH), 7.44 (d, *J* = 8.2 Hz, ArH), 7.30 (d, *J* = 7.8 Hz, ArH), 4.30-4.49 (m, Chiral-H), 3.28-3.40 (m, NHCH₂CH₂), 2.77 (s, NHCH₂CH₂), 2.66 (s, NCH₃), 1.53-1.90 (m, CH₂CH₂CH₃), 1.39 (s, Boc), 1.16-1.23 (m, CH₂CH₂CH₃), 0.88-0.89 (m, CH₂CH₂CH₃)

### Preparation Example 67: Preparation of (R)/(S)-tert-butyl methyl(1-oxo-1((2-(4'-(trifluoromethoxy)-[1,1'-biphenyl]-4-yl)ethyl)amino)hexan-2-yl)carbamate (72)

Compound 9 (0.74 g, 3.00 mmol), NMM (0.92 ml, 8.99 mmol), IBCF (0.51 ml, 3.90 mmol), and Compound 20 (1.00 g, 3.15 mmol) were reacted using Reaction Scheme e to synthesize Compound 72, (R)/(S)-tert-butyl methyl(1-oxo-1-((2-(4'-(trifluoromethoxy)-[1,1'-biphenyl]-4-yl)ethyl)amino)hexan-2-yl)carbamate (0.92 g, 60%) in the form of an oil..
R_{f} = 0.29 (EtOAc 1 : n-hexane 3);
¹H NMR (DMSO-*d₆*, 400 MHz) 7.88 (s, C(O)NH), 7.74-7.77 (m, ArH), 7.59 (d, *J* = 8.2 Hz, ArH), 7.44 (d, *J* = 8.0 Hz, ArH), 7.30 (d, *J* = 8.1 Hz, ArH), 4.28-4.47 (m, Chiral-H), 3.30-3.34 (m, NHCH₂CH₂), 2.77 (t, *J* = 6.7 Hz, NHCH₂CH₂), 2.66 (s, NCH₃), 1.53-1.72 (m, CH₂CH₂CH₂CH₃), 1.39 (s, Boc), 1.12-1.29 (m, CH₂CH₂CH₂CH₃), 0.84-0.90 (m, CH₂CH₂CH₂CH₃).

### Preparation Example 68: Preparation of (R)/(S)-2-amino-N-(2-(3',4'-dichloro-[1,1'-biphenyl]-4-yl)ethyl)butanamide hydrochloride (73)

Compound 54 (0.66 g, 1.47 mmol) and 4.0 M HCl (2.20 ml, 8.81 mmol in dioxane) were reacted using Reaction Scheme f to synthesize Compound 73, (R)/(S)-2-amino-N-(2-(3',4'-dichloro-[1,1'-biphenyl]-4-yl)ethyl)butanamide hydrochloride (0.52 g, 91%) in the form of a white powder.
R_{f} = 0.00 (EtOAc 9: acetone 1);
¹H NMR (DMSO-*d₆*, 400 MHz) 8.66 (t, *J* = 5.3 Hz, C(O)NH), 8.23 (s, NH₃), 7.92 (d, *J* = 1.9 Hz, ArH), 7.65-7.72 (m, ArH), 7.35 (d, *J* = 8.1 Hz, ArH), 3.67 (t, *J* = 6.0 Hz, Chiral-H), 3.31 - 3.55 (m, NHCH₂CH₂), 2.79-2.83 (m, NHCH₂CH₂), 1.67-1.74 (m, CH₂CH₃), 0.79 (t, *J* = 7.4 Hz, CH₂CH₃).

### Preparation Example 69: Preparation of (R)/(S)-2-amino-N-(2-(3',4'-dichloro-[1,1'-biphenyl]-4-yl)ethyl)pentanamide hydrochloride (74)

Compound 55 (0.95 g, 2.04 mmol) and 4.0 M HCl (3.06 ml, 12.2 mmol in dioxane) were reacted using Reaction Scheme f to synthesize Compound 74, (R)/(S)-2-amino-N-(2-(3',4'-dichloro-[1,1'-biphenyl]-4-yl)ethyl)pentanamide hydrochloride (0.66 g, 80%) in the form of a yellow powder.
R_{f} = 0.00 (EtOAc 9: acetone 1);
¹H NMR (DMSO-*d₆*, 400 MHz) 8.82 (t, *J* = 5.3 Hz, C(O)NH), 8.34 (s, NH₃), 7.64-7.90 (m, ArH), 7.35-7.38 (d, *J* = 8.1 Hz, ArH), 3.73 (t, *J* = 6.3 Hz, Chiral-H), 3.29-3.57 (m, NHCH₂CH₂), 2.82-2.85 (m, NHCH₂CH₂), 1.60-1.67 (m, CH₂CH₂CH₃), 1.14-1.22 (m, CH₂CH₂CH₃), 0.80 (t, *J* = 7.1 Hz, CH₂CH₂CH₃).

### Preparation Example 70: Preparation of (R)/(S)-2-amino-N-(2-(3',4'-dichloro-[1,1'-biphenyl]-4-yl)ethyl)hexanamide hydrochloride (75)

Compound 56 (0.75 g, 1.56 mmol) and 4.0 M HCl (2.35 ml, 9.39 mmol in dioxane) were reacted using Reaction Scheme f to synthesize Compound 75, (R)/(S)-2-amino-N-(2-(3',4'-dichloro-[11'-biphenyl]-4-yl)ethyl)hexanamide hydrochloride (0.60 g, 92%) in the form of a white powder.
R_{f} = 0.00 (EtOAc 9: acetone 1);
¹H NMR (DMSO-*d₆*, 400 MHz) 8.60 (t, *J* = 5.5 Hz, C(O)NH), 8.18 (s, NH ₃), 7.91 (d, *J* = 2.0 Hz, ArH), 7.65-7.72 (m, ArH), 7.35 (d, *J* = 8.2 Hz, ArH), 3.66 (t, *J* = 6.2 Hz, Chiral-H), 3.28-3.58 (m, NHCH₂CH₂), 2.81-2.85 (m, NHCH₂CH₂), 1.59-1.64 (m, CH₂CH₂CH₂CH₃), 1.14-1.23 (m, CH₂CH₂CH₂CH₃), 0.79 (t, *J* = 6.8 Hz, CH₂CH₂CH₂CH₃).

### Preparation Example 71: Preparation of (R)/(S)-2-amino-N-(2-(4'-(trifluoromethyl)-[1,1'-biphenyl]-4-yl)ethyl)butanamide hydrochloride (76)

Compound 57 (0.90 g, 2.00 mmol) and 4.0 M HCl (3.00 ml, 12.0 mmol in dioxane) were reacted using Reaction Scheme f to synthesize Compound 76, (R)/(S)-2-amino-N-(2-(4'-(trifluoromethyl)-[1,1'-biphenyl]-4-yl)ethyl)butanamide hydrochloride (0.75 g, 96%) in the form of a white powder.
R_{f} = 0.00 (EtOAc 9: acetone 1);
¹H NMR (DMSO-*d₆*, 400 MHz) 8.62 (s, C(O)NH), 8.17 (s, NH ₃), 7.88 (d, *J* = 8.2 Hz, ArH), 7.81 (d, J = 8.4 Hz, ArH), 7.68 (d, J = 8.3 Hz, ArH), 7.38 (d, *J* = 8.1 Hz, ArH), 3.66 (t, *J* = 6.1 Hz, Chiral-H), 3.30-3.57 (m, NHCH₂CH₂), 2.82 (t, *J* = 7.0 Hz, NHCH₂CH₂), 1.67-1.74 (m, CH₂CH₃), 0.79 (t, *J* = 7.5 Hz, CH₂CH₃).

### Preparation Example 72: Preparation of (R)/(S)-2-amino-N-(2-(4'-(trifluoromethyl)-[1,1'-biphenyl]-4-yl)ethyl)pentanamide hydrochloride (77)

Compound 58 (1.03 g, 2.22 mmol) and 4.0 M HCl (5.56 ml, 22.2 mmol in dioxane) were reacted using Reaction Scheme f to synthesize Compound 77, (R)/(S)-2-amino-N-(2-(4'-(trifluoromethyl)-[1,1'-biphenyl]-4-yl)ethyl)pentanamide hydrochloride (0.75 g, 85%) in the form of a white powder.
R_{f} = 0.00 (EtOAc 9: acetone 1);
¹H NMR (DMSO-*d₆*, 300 MHz) 8.81 (t, *J* = 5.3 Hz, C(O)NH), 8.35 (s, NH₃), 7.84 (dd, *J* = 8.2 Hz, 15.7 Hz, ArH), 7.54 (dd, *J* = 8.1 Hz, 73.4 Hz, ArH), 3.71-3.75 (t, J = 6.3 Hz, Chiral-H), 3.30-3.60 (m, NH₂CH₂CH₂), 2.85 (t, *J* = 6.3 Hz, NHCH₂CH₂), 1.61-1.68 (m, CH₂CH₂CH₃), 1.15-1.22 (m, CH₂CH₂CH₃), 0.80 (t, *J* = 7.2 Hz, CH₂CH₂CH₃).

### Preparation Example 73: Preparation of (R)/(S)-2-amino-N-(2-(4'-(trifluoromethyl)-[1,1'-biphenyl]-4-yl)ethyl)hexanamide hydrochloride (78)

Compound 59 (1.32 g, 2.76 mmol) and 4.0 M HCl (4.14 ml, 16.6 mmol in dioxane) were reacted using Reaction Scheme f to synthesize Compound 78, (R)/(S)-2-amino-N-(2-(4'-(trifluoromethyl)-[1,1'-biphenyl]-4-yl)ethyl)hexanamide hydrochloride (0.82 g, 72%) in the form of a white powder.
R_{f} = 0.00 (EtOAc 9: acetone 1);
¹H NMR (DMSO-*d₆*, 400 MHz) 8.69 (t, *J* = 4.9 Hz, C(O)NH), 8.26 (s, NH₃), 7.88 (d, *J* = 8.2 Hz, ArH), 7.81 (d, *J* = 8.3 Hz, ArH), 7.68 (d, *J* = 8.1 Hz, ArH), 7.39 (d, *J* = 8.0 Hz, ArH), 3.69 (t, *J* = 6.2 Hz, Chiral-H), 3.29-3.59 (m, NHCH₂CH₂), 2.80-2.89 (m, NHCH₂CH₂), 1.61-1.67 (m, CH₂CH₂CH₂CH₃), 1.14-1.23 (m, CH₂CH₂CH₂CH₃), 0.79 (t, *J* = 6.8 Hz, CH₂CH₂CH₂CH₃).

### Preparation Example 74: Preparation of (R)/(S)-2-amino-N-(2-(4'-(trifluoromethoxy)-[1,1'-biphenyl]-4-yl)ethyl)butanamide hydrochloride (79)

Compound 60 (0.91 g, 1.95 mmol) and 4.0 M HCl (2.92 ml, 11.7 mmol in dioxane) were reacted using Reaction Scheme f to synthesize Compound 79, (R)/(S)-2-amino-N-(2-(4'-(trifluoromethoxy)-[1,1'-biphenyl]-4-yl)ethyl)butanamide hydrochloride (0.43 g, 54%) in the form of a yellow powder.
R_{f} = 0.00 (EtOAc 9: acetone 1);
¹H NMR (DMSO-*d₆*, 400 MHz) 8.67 (s, C(O)NH), 8.24 (s, NH₃), 7.35-7.89 (m, ArH), 3.67 (d, *J* = 4.3 Hz, Chiral-H), 3.30-3.55 (m, NHCH₂CH₂), 2.80-2.85 (m, NHCH₂CH₂), 1.68-1.75 (m, CH₂CH₃), 0.80 (t, *J* = 7.5 Hz, CH₂CH₃).

### Preparation Example 75: Preparation of (R)/(S)-2-amino-N-(2-(4'-(trifluoromethoxy)-[1,1'-biphenyl]-4-yl)ethyl)pentanamide hydrochloride (80)

Compound 61 (0.70 g, 1.45 mmol) and 4.0 M HCl (2.18 ml, 8.70 mmol in dioxane) were reacted using Reaction Scheme f to synthesize Compound 80, (R)/(S)-2-amino-N-(2-(4'-(trifluoromethoxy)-[1,1'-biphenyl]-4-yl)ethyl)pentanamide hydrochloride (0.55 g, 91%) in the form of a white powder.
R_{f} = 0.00 (EtOAc 9: acetone 1);
¹H NMR (DMSO-*d₆*, 400 MHz) 8.71 (t, *J* = 5.4 Hz, C(O)NH), 8.28 (s, NH ₃), 7.77 (d, *J* = 8.8 Hz, ArH), 7.62 (d, *J* = 8.2 Hz, ArH), 7.35-7.45 (m, ArH), 3.70 (t, *J* = 6.4 Hz, Chiral-H), 3.28-3.57 (m, NHCH₂CH₂), 2.80-2.86 (m, NHCH₂CH₂), 1.61-1.65 (m, CH₂CH₂CH₃), 1.13-1.24 (m, CH₂CH₂CH₃), 0.80 (t, *J* = 7.4 Hz, CH₂CH₂CH₃).

### Preparation Example 76: Preparation of (R)/(S)-2-amino-N-(2-(4'-(trifluoromethoxy)-[1,1'-biphenyl]-4-yl)ethyl)hexanamide hydrochloride (81)

Compound 62 (0.79 g, 1.59 mmol) and 4.0 M HCl (2.38 ml, 9.54 mmol in dioxane) were reacted using Reaction Scheme f to synthesize Compound 81, (R)/(S)-2-amino-N-(2-(4'-(trifluoromethoxy)-[1,1'-biphenyl]-4-yl)ethyl)hexanamide hydrochloride (0.62 g, 91%) in the form of a white powder.
R_{f} = 0.00 (EtOAc 9: acetone 1);
¹H NMR (DMSO-*d₆*, 400 MHz) 8.66 (t, *J* = 5.4 Hz, C(O)NH), 8.23 (s, NH₃), 7.88 (d, *J* = 8.2 Hz, ArH), 7.81 (d, *J* = 8.4 Hz, ArH), 7.68 (d, *J* = 8.2 Hz, ArH), 7.39 (d, *J* = 8.2 Hz, ArH), 3.68 (t, *J* = 6.4 Hz, Chiral-H), 3.29-3.59 (m, NHCH₂CH₂), 2.81-2.87 (m, NHCH₂CH₂), 1.60-1.66 (m, CH₂CH₂CH₂CH₃), 1.13-1.23 (m, CH₂CH₂CH₂CH₃), 0.79 (t, *J* = 7.0 Hz, CH₂CH₂CH₂CH₃).

### Preparation Example 77: Preparation of (R)/(S)-2-amino-N-(2-(3',4'-difluoro-[1,1'-biphenyl]-4-yl)ethyl)pentanamide hydrochloride (82)

Compound 62 (0.94 g, 2.17 mmol) and 4.0 M HCl (3.26 ml, 13.0 mmol in dioxane) were reacted using Reaction Scheme f to synthesize Compound 82, (R)/(S)-2-amino-N-(2-(3',4'-difluoro-[1 ,1'-biphenyl]-4-yl)ethyl)pentanamide hydrochloride (0.68 g, 85%) in the form of a white powder.
R_{f} = 0.00 (EtOAc 9: acetone 1);
¹H NMR (DMSO-*d₆*, 400 MHz) 8.55 (s, C(O)NH), 8.10 (s, NH₃), 7.72-7.77 (m, ArH), 7.62 (d, *J* = 8.1 Hz, ArH), 7.50-7.53 (m, ArH), 7.33 (d, *J* = 8.1 Hz, ArH), 3.65 (t, *J* = 5.9 Hz, Chiral-H), 3.29-3.57 (m, NHCH₂CH₂), 2.78-2.84 (m, NHCH₂CH₂), 1.58 (q, *J* = 6.9 Hz, 8.8 Hz, CH₂CH₂CH₃), 1.14-1.20 (m, CH₂CH₂CH₃), 0.80 (t, *J* = 7.3 Hz, CH₂CH₂CH₃).

### Preparation Example 78: Preparation of (R)/(S)-N-(2-(3',4'-dichloro-[1,1'-biphenyl]-4-yl)ethyl)-2-(methylamino)butanamide hydrochloride (83)

Compound 64 (1.31 g, 2.81 mmol) and 4.0 M HCl (4.20 ml, 16.9 mmol in dioxane) were reacted using Reaction Scheme f to synthesize Compound 83, (R)/(S)-N-(2-(3',4'-dichloro-[1,1'-biphenyl]-4-yl)ethyl)-2-(methylamino)butanamide hydrochloride (1.07 g, 94%) in the form of a yellow powder.
R_{f} = 0.00 (EtOAc 9: acetone 1);
¹H NMR (DMSO-*d₆*, 400 MHz) 8.79 (s, C(O)NH, NH₂), 7.92 (d, *J* = 2.0 Hz, ArH), 7.65-7.72 (m, ArH), 7.36 (d, *J* = 8.1 Hz, ArH), 3.59 (t, *J* = 6.4 Hz, Chiral-H), 3.39-3.53 (m, NHCH₂CH₂), 2.83 (t, *J* = 6.8 Hz, NHCH₂CH₂), 2.38 (t, *J* = 6.1 Hz, NCH₃), 1.68-1.80 (m, CH₂CH₃), 0.77 (t, *J* = 7.5 Hz, CH₂CH₃).

### Preparation Example 79: Preparation of (R)/(S)-N-(2-(3',4'-dichloro-[1,1'-biphenyl]-4-yl)ethyl)-2-(methylamino)pentanamide hydrochloride (84)

Compound 65 (0.65 g, 1.36 mmol) and 4.0 M HCl (2.03 ml, 8.13 mmol in dioxane) were reacted using Reaction Scheme f to synthesize Compound 84, (R)/(S)-N-(2-(3',4'-dichloro-[1,1'-biphenyl]-4-yl)ethyl)-2-(methylamino)pentanamide hydrochloride (0.33 g, 57%) in the form of a yellow powder.
R_{f} = 0.00 (EtOAc 9: acetone 1);
¹H NMR (DMSO-*d₆*, 400 MHz) 9.26 (s, NH₂), 8.96 (t, *J* = 5.3 Hz, C(O)NH), 7.90-7.91 (m, ArH), 7.64-7.71 (m, ArH), 7.36 (d, *J* = 8.1 Hz, ArH), 3.64-3.68 (m, Chiral-H), 3.42-3.56 (m, NHCH₂CH₂), 2.84 (t, *J* = 6.8 Hz, NHCH₂CH₂), 2.35 (s, NCH₃), 1.60-1.77 (m, CH₂CH₂CH₃), 1.09-1.16 (m, CH₂CH₂CH₃), 0.80 (t, *J* = 7.2 Hz, CH₂CH₂CH₃).

### Preparation Example 80: Preparation of (R)/(S)-N-(2-(3',4'-dichloro-[1,1'-biphenyl]-4-yl)ethyl)-2-(methylamino)hexanamide hydrochloride (85)

Compound 66 (0.57 g, 1.16 mmol) and 4.0 M HCl (1.74 ml, 6.95 mmol in dioxane) were reacted using Reaction Scheme f to synthesize Compound 85, (R)/(S)-N-(2-(3',4'-dichloro-[1,1'-biphenyl]-4-yl)ethyl)-2-(methylamino)hexanamide hydrochloride (0.38 g, 75%) in the form of a white powder.
R_{f} = 0.00 (EtOAc 9: acetone 1);
¹H NMR (DMSO-*d₆*, 400 MHz) 8.70-9.36 (s, NH₂), 8.80 (t, *J* = 5.4 Hz, C(O)NH), 7.91 (d, *J* = 2.0 Hz, ArH), 7.65-7.72 (m, ArH), 7.35 (d, *J* = 8.2 Hz, ArH), 3.55 (t, *J* = 6.6 Hz, Chiral-H), 3.38-3.52 (m, NHCH₂CH₂), 2.83 (t, *J* = 6.8 Hz, NHCH₂CH₂), 2.35 (s, NCH₃), 1.60-1.76 (m, CH₂CH₂CH₂CH₃), 1.05-1.23 (m, CH₂CH₂CH₂CH₃), 0.78 (t, *J* = 7.2 Hz, CH₂CH₂CH₂CH₃).

### Preparation Example 81: Preparation of (R)/(S)-2-(methylamino)-N-(2-(4'-(trifluoromethyl)-[1,1'-biphenyl]-4-yl)ethyl)butanamide hydrochloride (86)

Compound 67 (0.86 g, 1.84 mmol) and 4.0 M HCl (2.77 ml, 11.1 mmol in dioxane) were reacted using Reaction Scheme f to synthesize Compound 86, (R)/(S)-2-(methylamino)-N-(2-(4'-(trifluoromethyl)-[1,1'-biphenyl]-4-yl)ethyl)butanamide hydrochloride (0.65 g, 88%) in the form of a white powder.
R_{f} = 0.00 (EtOAc 9: acetone 1);
¹H NMR (DMSO-*d₆*, 400 MHz) 9.04 (s, NH₂), 8.79 (s, C(O)NH), 7.87 (d, *J* = 8.3 Hz, ArH), 7.80 (d, *J* = 8.4 Hz, ArH), 7.68 (d, *J* = 8.2 Hz, ArH), 7.39 (d, *J* = 8.2 Hz, ArH), 3.59 (t, *J* = 2.5 Hz, 4.9 Hz, Chiral-H), 3.40-3.56 (m, NHCH₂CH₂), 2.84 (t, *J* = 6.9 Hz, NHCH₂CH₂), 2.37 (s, NCH₃), 1.67-1.85 (m, CH₂CH₃), 0.77 (t, *J* = 7.5 Hz, CH₂CH₃).

### Preparation Example 82: Preparation of (R)/(S)-2-(methylamino)-N-(2-(4'-(trifluoromethyl)-[1,1'-biphenyl]-4-yl)ethyl)pentanamide hydrochloride (87)

Compound 68 (1.17 g, 2.45 mmol) and 4.0 M HCl (3.67 ml, 14.7 mmol in dioxane) were reacted using Reaction Scheme f to synthesize Compound 87, (R)/(S)-2-(methylamino)-N-(2-(4'-(trifluoromethyl)-[1,1'-biphenyl]-4-yl)ethyl)pentanamide hydrochloride (81%) in the form of a white powder.
R_{f} = 0.00 (EtOAc 9: acetone 1);
¹H NMR (DMSO-*d₆*, 400 MHz) 8.93-9.79 (m, NH₂), 9.04 (t, *J* = 5.2 Hz, C(O)NH), 7.84 (dd, *J* = 8.3 Hz, 15.1 Hz, ArH), 7.54 (dd, *J* = 8.0 Hz, 73.4 Hz, ArH), 3.68-3.72 (m, Chiral-H), 3.42-3.58 (m, NHCH₂CH₂), 2.87 (t, *J* = 6.7 Hz, NHCH₂CH₂), 2.36 (s, NCH₃), 1.64-1.82 (m, CH₂CH₂CH₃), 1.09-1.22 (m, CH₂CH₂CH₃), 0.81 (t, J = 7.1 Hz, CH₂CH₂CH₃).

### Preparation Example 83: Preparation of (R)/(S)-2-(methylamino)-N-(2-(4'-(trifluoromethyl)-[1,1'-biphenyl]-4-yl)ethyl)hexanamide hydrochloride (88)

Compound 69 (0.85 g, 1.72 mmol) and 4.0 M HCl (2.60 ml, 10.3 mmol in dioxane) were reacted using Reaction Scheme f to synthesize Compound 88, (R)/(S)-2-(methylamino)-N-(2-(4'-(trifluoromethyl)-[1,1'-biphenyl]-4-yl)ethyl)hexanamide hydrochloride (0.53 g, 71%) in the form of a white powder.
R_{f} = 0.00 (EtOAc 9: acetone 1);
¹H NMR (DMSO-*d₆*, 400 MHz) 9.00 (s, NH₂), 8.75 (t, *J* = 5.4 Hz, C(O)NH), 7.87 (d, *J* = 8.3 Hz, ArH), 7.81 (d, *J* = 8.4 Hz, ArH), 7.68 (d, *J* = 8.2 Hz, ArH), 7.38 (d, *J* = 8.2 Hz, ArH), 3.57-3.61 (m, Chiral-H), 3.40-3.55 (m, NHCH₂CH₂), 2.84 (t, *J* = 6.8 Hz, NHCH₂CH₂), 2.36 (s, NCH₃), 1.60-1.75 (m, CH₂CH₂CH₂CH₃), 1.06-1.24 (m, CH₂CH₂CH₂CH₃), 0.78 (t, *J* = 7.2 Hz, CH₂CH₂CH₂CH₃).

### Preparation Example 84: Preparation of (R)/(S)-2-(methylamino)-N-(2-(4'-(trifluoromethoxy)-[1,1'-biphenyl]-4-yl)ethyl)butanamide hydrochloride (89)

Compound 70 (0.97 g, 2.01 mmol) and 4.0 M HCl (3.00 ml, 12.1 mmol in dioxane) were reacted using Reaction Scheme f to synthesize Compound 89, (R)/(S)-2-(methylamino)-N-(2-(4'-(trifluoromethoxy)-[1,1'-biphenyl]-4-yl)ethyl)butanamide hydrochloride (0.73 g, 88%) in the form of a yellow powder.
R_{f} = 0.00 (EtOAc 9: acetone 1);
¹H NMR (DMSO-*d₆*, 400 MHz) 9.00 (s, NH₂), 8.77 (s, C(O)NH), 7.77 (d, *J* = 8.7 Hz, ArH), 7.61 (d, *J* = 8.2 Hz, ArH), 7.44 (d, *J* = 8.2 Hz, ArH), 7.35 (d, *J* = 8.1 Hz, ArH), 3.57-3.60 (m, Chiral-H), 3.39-3.55 (m, NHCH₂CH₂), 2.82 (t, *J* = 6.9 Hz, NHCH₂CH₂), 2.37 (s, NCH₃), 1.67-1.83 (m, CH₂CH₃), 0.77 (t, *J* = 7.5 Hz, CH₂CH₃).

### Preparation Example 85: Preparation of (R)/(S)-2-(methylamino)-N-(2-(4'-(trifluoromethoxy)-[1,1'-biphenyl]-4-yl)ethyl)pentanamide hydrochloride (90)

Compound 71 (0.83 g, 1.67 mmol) and 4.0 M HCl (2.51 ml, 10.0 mmol in dioxane) were reacted using Reaction Scheme f to synthesize Compound 90, (R)/(S)-2-(methylamino)-N-(2-(4'-(trifluoromethoxy)-[1,1'-biphenyl]-4-yl)ethyl)pentanamide hydrochloride (0.40 g, 61%) in the form of a white powder.
R_{f} = 0.00 (EtOAc 9: acetone 1);
¹H NMR (DMSO-*d₆*, 400 MHz) 8.85-9.19 (m, NH₂), 8.75 (s, C(O)NH), 7.76 (d, *J* = 7.7 Hz, ArH), 7.61 (d, *J* = 7.5 Hz, ArH), 7.44 (d, *J* = 8.0 Hz, ArH), 7.35 (d, *J* = 7.4 Hz, ArH), 3.60 (s, Chiral-H), 3.40-3.56 (m, NHCH₂CH₂), 2.83 (t, *J* = 6.5 Hz, NHCH₂CH₂), 2.36 (s, NCH₃), 1.60-1.66 (m, CH₂CH₂CH₃), 1.09-1.17 (m, CH₂CH₂CH₃), 0.80 (t, *J* = 7.0 Hz, CH₂CH₂CH₃).

### Preparation Example 86: Preparation of (R)/(S)-2-(methylamino)-N-(2-(4'-(trifluoromethoxy)-[1,1'-biphenyl]-4-yl)ethyl)hexanamide hydrochloride (91)

Compound 72 (0.89 g, 1.74 mmol) and 4.0 M HCl (2.61 ml, 10.4 mmol in dioxane) were reacted using Reaction Scheme f to synthesize Compound 91, (R)/(S)-2-(methylamino)-N-(2-(4'-(trifluoromethoxy)-[1,1'-biphenyl]-4-yl)ethyl)hexanamide hydrochloride (0.66 g, 86%) in the form of a white powder.
R_{f} = 0.00 (EtOAc 9: acetone 1);
¹H NMR (DMSO-*d₆*, 400 MHz) 8.95 (s, NH₂), 8.72 (s, C(O)NH), 7.76 (d, *J* = 8.7 Hz, ArH), 7.61 (d, *J* = 8.1 Hz, ArH), 7.44 (d, *J* = 8.4 Hz, ArH), 7.35 (d, *J* = 8.1 Hz, ArH), 3.57-3.60 (m, Chiral-H), 3.29-3.56 (m, NHCH₂CH₂), 2.83 (t, *J* = 6.8 Hz, NHCH₂CH₂), 2.37 (s, NCH₃), 1.59-1.74 (m, CH₂CH₂CH₂CH₃), 1.08-1.24 (m, CH₂CH₂CH₂CH₃), 0.78 (t, *J* = 7.2 Hz, CH₂CH₂CH₂CH₃).

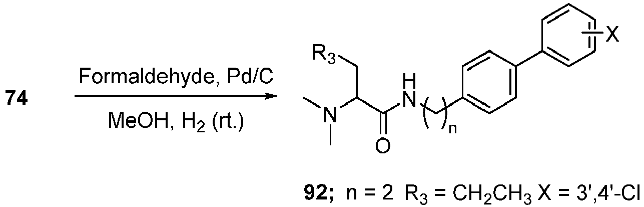

### Preparation Example 87: Preparation of (R)/(S)-N-(2-(3',4'-dichloro-[1,1'-biphenyl]-4-yl)ethyl)-2-(dimethylamino)pentanamide (92)

Compound 74 (1.0 eq), triethylamine (6.0 eq), formaldehyde (1.05 eq), and a palladium catalyst (0.2 eq) were reacted using Reaction Scheme g to synthesize Compound 92, (R)/(S)-N-(2-(3',4'-dichloro-[1,1'-biphenyl]-4-yl)ethyl)-2-(dimethylamino)pentanamide.

¹H NMR (DMSO-*d₆*, 400 MHz) 8.96 (t, *J* = 5.3 Hz, C(O)NH), 7.90-7.91 (m, ArH), 7.64-7.71 (m, ArH), 7.36 (d, *J* = 8.1 Hz, ArH), 3.64-3.68 (m, Chiral-H), 3.42-3.56 (m, NHCH₂CH₂), 2.82-2.85 (m, NHCH₂CH₂), 2.35 (s, N(CH₃)₂), 1.60-1.77 (m, CH₂CH₂CH₃), 1.09-1.16 (m, CH₂CH₂CH₃), 0.80 (t, *J* = 7.2 Hz, CH₂CH₂CH₃).

### Example

### Example 1: Cell Culture

Mouse macrophage cell line Raw264.7 (obtained from the Korean Cell Line Bank) and HaCaT keratinocyte cell line (obtained from Addexbio, T0020001) were cultured in Dulbecco's modified Eagle's medium (DMEM, Gibco Cat No. 11995073) supplemented with 10% fetal bovine serum (FBS, Gibco, Cat No. 26140-079) and 10,000 U/mL penicillin-streptomycin (Gibco, Cat No. 15140-122). The cells were cultured in a CO₂ incubator (Thermo Scientific, Cat No. 51023121) at 37 °C temperature.

### Example 2: Induction of Interleukin-6 (IL-6) in RAW264.7 Cells to Evaluate the Anti-inflammatory Effects of Compounds

### (1) Induction of IL-6 production using LPS

The inhibitory effect on induced IL-6 cytokine production was analyzed. RAW264.7 cells (obtained from the Korean Cell Line Bank) were seeded at a density of 5 × 10⁵ cells/well in a 12-well plate and stabilized in a CO₂ incubator at 37 °C with 5% carbon dioxide and saturated humidity. After 24 hours of incubation, the compounds of the present invention were prepared in DMEM at concentrations of 0 µg/mL or 8 µg/mL and pre-treated for 30 minutes. Next, lipopolysaccharide (LPS, Sigma-Aldrich, Cat No. L2887) was treated to a concentration of 1 µg/mL to induce an inflammatory response for 24 hours. Subsequently, mouse IL-6 ELISA kit (Labiskoma, Cat No. K0331230P) and mouse TNF-α ELISA kit (Labiskoma, Cat No. K0331186P) were used according to the manufacturer's instructions, and absorbance was measured. FK506 (FK-506 monohydrate, manufacturer: MilliporeSigma, product number: F4679, U.S.A) was used as a control substance.

### (2) Induction of IL-6 Production Using TNF-α or IFN-β

TNF-α (Sigma-Aldrich, Cat No. T6674) or IFN-β (Sino Biological, Cat No. 10704-HNAS) was treated at a concentration of 50 ng/mL each to induce an inflammatory response for 24 hours. The control substance and compounds of the present invention were each pre-treated for 1 hour. The remaining steps were performed in the same manner as described in (1) above.

### (3) IL-6 analysis

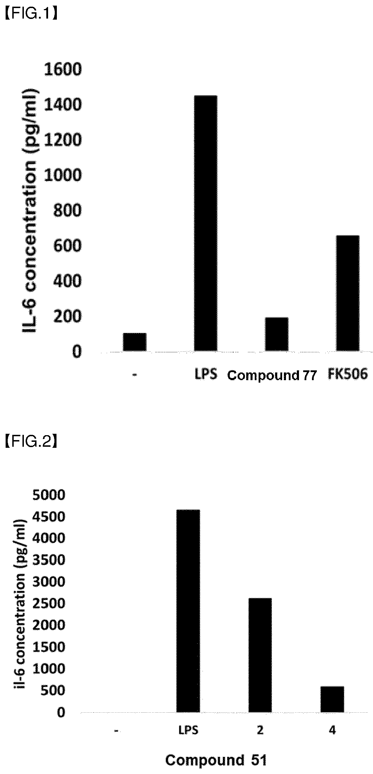

After inducing inflammation as described in (1) and (2) above, the supernatant was collected, and the quantification of IL-6 in the supernatant was performed using the IL-6 ELISA kit (Mouse IL-6 ELISA Kit, pink-ONE, manufactured by Komabiotech, Product No. K0331230P) according to the manufacturer's instructions. Specifically, the amount of IL-6 in the supernatant was quantified according to the ELISA kit protocol, and the absorbance was measured at OD 450 nm using a microplate reader (Multiskan Sky, manufactured by Thermo Fisher Scientific). The inhibitory activity of the compounds of the present invention on IL-6 production induced by LPS treatment was shown in Table 1 to Table 3 and Figure 1 to Figure 2. Table 1 and Table 2 below show the results comparing the amount of IL-6 production with the control drug after LPS treatment for the compounds 77 and 51 of the present invention, respectively. Table 3 represents the results comparing the amount of IL-6 production after LPS treatment between the compounds of the present invention and the control compound. In this example, it was confirmed that the treatment with the compounds of the present invention significantly reduced the amount of IL-6 production compared to the groups treated with LPS, TNF-α, or IFN-β. In Table 1 to Table 3, Figure 1, and Figure 2, the relative values of IL-6 generated after pretreatment with the compounds of the present invention were expressed as a percentage relative to the IL-6 production (100%) induced by LPS, TNF-α, or IFN-β treatment.

**[Table 1]**

| | 1 well | 2 well | IL-6 (pg/mℓ) | IL-6 (%) |
|---|---|---|---|---|
| - | 0.10 | 0.10 | 102.62 | 7.08 |
| LPS | 1.02 | 0.91 | 1449.82 | 100.00 |
| compound 77 (8 *µ*g/mℓ) | 0.18 | 0.15 | 191.20 | 13.19 |
| FK506 | 0.49 | 0.47 | 656.86 | 45.31 |

**[Table 2]**

| | 1 well | 2 well | std | avg | avg (%) |
|---|---|---|---|---|---|
| - | 0 | 0 | 0 | 0 | 0.00 |
| LPS | 4628.00 | 4695.00 | 47.38 | 4661.50 | 100.00 |
| compound 51 (2 *µ*g/ml) | 2815.00 | 2416.00 | 282.14 | 2615.50 | 56.11 |
| compound 51 (4 *µ*g/ml) | 669.30 | 504.60 | 116.46 | 586.95 | 12.59 |

**[Table 3]**

| Compound number | Relative amount of IL-6 compared to LPS induction (%) |
|---|---|
| 76 | 9.58 |
| 47 | 12 |
| 48 | 67 |
| 49 | 50 |
| 50 | 68 |
| 52 | 13 |
| 32 | 16 |
| 53 | 5 |
| 77 | 5 |
| 45 | 3 |
| 80 | 6 |
| 82 | 3 |
| 83 | 9 |
| 36 | 9 |
| 87 | 24 |

### Example 3: Anti-inflammatory Effects of Compound 77 in a HaCaT Keratinocyte cell line

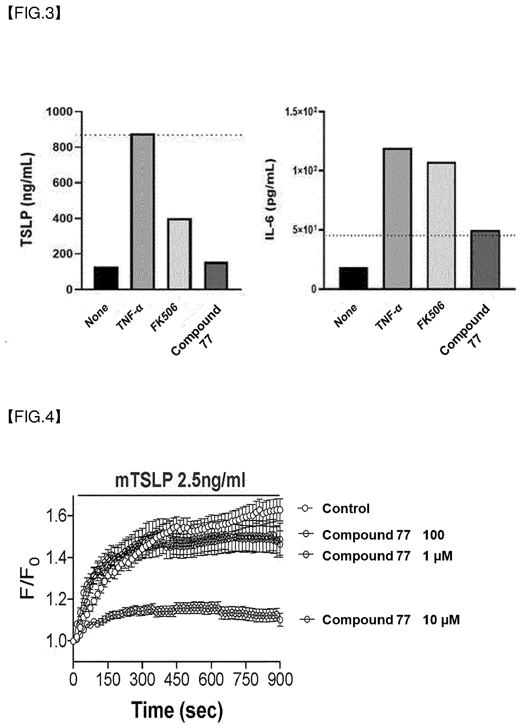

HaCaT keratinocytes, a human skin keratinocyte cell line (obtained from the Korean Cell Line Bank), were seeded in a 12-well plate at a density of 1 × 10⁵ cells per well and cultured for at least 12 hours in a 37°C humidified incubator containing 5% carbon dioxide and saturated water vapor. After pre-treatment with the compound 77 of the present invention (0 µg/ml or 8 µg/ml) or the immunosuppressant FK506 (FK-506 monohydrate, manufacturer: MilliporeSigma product number: F4679, USA) as a control for 1 hour, TNF-α (Tumor Necrosis Factor-α human, manufacturer: MilliporeSigma) was treated for 24 hours as an inflammatory response inducer at a concentration of 100 ng/m2. Next, the supernatant was obtained and TSLP and IL-6 ELISA (Human TSLP and IL-6 ELISA Kit, pink-ONE, manufacturer: Komabiotech) each was performed. Specifically, the contents of TSLP and IL-6 in the supernatant were quantified according to the protocol of the ELISA kit with the supernatant, and absorbance was measured at a wavelength of 450 nm using a microplate reader (Multiskan Sky, manufacturer: Thermo Fisher Scientific, product number: 51119600DP). Fig. 3 is a result of confirming the amount of TSLP and interleukin-6 according to the embodiment of the present invention. As shown in Fig. 3, when the compound 77 of the present invention was treated, it was confirmed that the amounts of TSLP and interleukin-6 were significantly reduced compared to the control group FK506, thereby confirming anti-inflammatory effects of the compound 77.

### Example 4: Confirmation of TSLP and TSLPR interaction inhibition

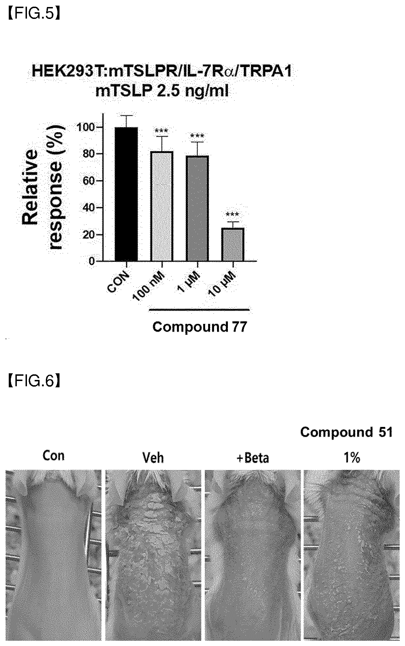

Ca2+ influx by TSLP was detected using HEK293T cells, and whether the compound of the present invention inhibits the interaction between TSLP and TSLPR was analyzed. HEK293T cells transiently transfected with three genes (mouse TSLPR, IL-7Rα, TRPA1, Professor Won-Sik Shim, Gachon University, Korea) were employed in this experiment. For the measurement of Ca2+ influx, a calcium-specific fluorescent dye Fluo3-AM (Sigma-Aldrich) was used. Mouse TSLP (2.5 ng/mL) was applied and fluorescence intensity was monitored up to 15 min. For comparison, compound 77 of the present invention (0.1 nM to 10 µM) was pre-treated for 10 minutes before TSLP treatment. Figs. 4 and 5 show the results. Responses represent maximum fluorescence intensity at the first time point (0 min).

### Experimental Example: Confirmation of efficacy of the compound of the present invention in mouse psoriasis model

### Preparation of test articles and mouse model manufacturing

After dissolving the test substance (Compound 51 of the present invention) using DMSO (Dimethyl sulfoxide, Sigma, Cat No. 472301), an appropriate concentration was prepared with PBS (HyClone, Cat No. SH30028.02). The transdermal test substance was prepared by dissolving the test substance in 100% ethanol (Medchem, Cat No. HY-13571). Betamethasone dipropionate (Sigma, Cat No.D1756, Lot No.BCBW7684), a positive control, was dissolved in DMSO, and PEG400 (Sigma, Cat.No. 06855) and 30% ethanol (Daejung, Cat.No. 4023-4100) were added to the mixture obtained above. The same vehicle was used for the negative control in the same way as the positive control specimen preparation.

BALB/c mice (purchased through JA BIO, 27 males, 8 weeks old) were acclimatized for one week. After the mice were anesthetized with isoflurane 3 days before the application of Aldara cream, the hair on the back (3 × 3 cm) was removed using a clipper. The remaining hair was completely removed by leaving the hair removal cream for 1 minute. The mice were left for 72 hours to heal the wounds. To induce psoriasis, Aldera Cream (5% Imiquimod, Dong-A ST) 62.5 mg was applied at the same time point (AM 10:00) once/day 7 times, and 150 µl of test substance or positive control was applied from the 4th day at the same time point (PM 14:00) for 4 days. The composition of each test group was as follows. a) Con: Normal + excipient application group: 8 animals; b) Veh: Aldara cream + excipient application group: 8 animals; c) Beta: Aldara + Betamethasone 0.05% applied group: 8 animals; and d) Compound 51 1%: Aldara + Compound 51 1% application group: 3 animals. The autopsy proceeded in a non-fasting state, and the dorsal skin was removed from each mouse, rapidly frozen in liquid nitrogen, and stored at -80 °C before for the subsequent experiments.

### Efficacy evaluation and analysis of results

### (1) Observation of general symptoms

All animals were observed at least once a day for death, type of general symptoms, date of onset of symptom, and severity of symptoms during the administration and observation period.

### (2) PASI (Psoriasis area and severity index) score

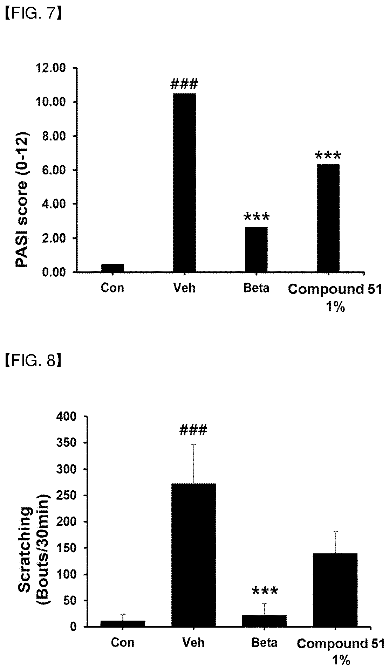

After taking pictures on the 0th, 4th, and 8th days to measure the skin index values, they were visually observed and recorded to measure the degree of skin lesions on the dorsal area. The condition of psoriasis skin lesions in each group was scored according to the evaluation index (erythema, skin thickness, dead skin cells) as None (0), Weak (1), Moderate (2), Severe (3), Very Severe (4) and set the score, and by summing up the scores of the three items, an evaluation score between a minimum of 0 and a maximum of 12 was calculated. When comparing the pictures on the 8th day, it was confirmed that psoriasis was induced in the Veh group compared to Con, and that the skin lesions were visually improved in the Beta and KDS6002 treated groups. Fig. 6 shows the results. After taking pictures in all groups on the 0th, 4th and 8th days, the evaluation indexes (erythema, skin thickness, and dead skin cells) of the psoriasis skin lesions in each group were graphed. It was confirmed that the PASI Score increased in the Veh group compared to Con and significantly decreased in the Beta and Compound 51 treated groups (p<0.001, see Fig. 7).

### (3) Evaluation of scratching

On the day of autopsy, each subject in the test group had an adaptation time for 1 hour in a space without stimulation, and then video was taken for 30 minutes, and then scratching counts were measured for the sensitized area. Changes in pruritus by the administration of Compound 51 of the present invention were compared with the Veh. In the evaluation of pruritus measured on the 8th day, a significant increase was confirmed in the Veh group and a significant decrease in the Beta group. A tendency to decrease pruritus was confirmed in the Compound 51 treatment group of the present invention. The results are shown in Fig. 8.

### (4) Quantitative RT-PCR (qRT-PCR) measurement

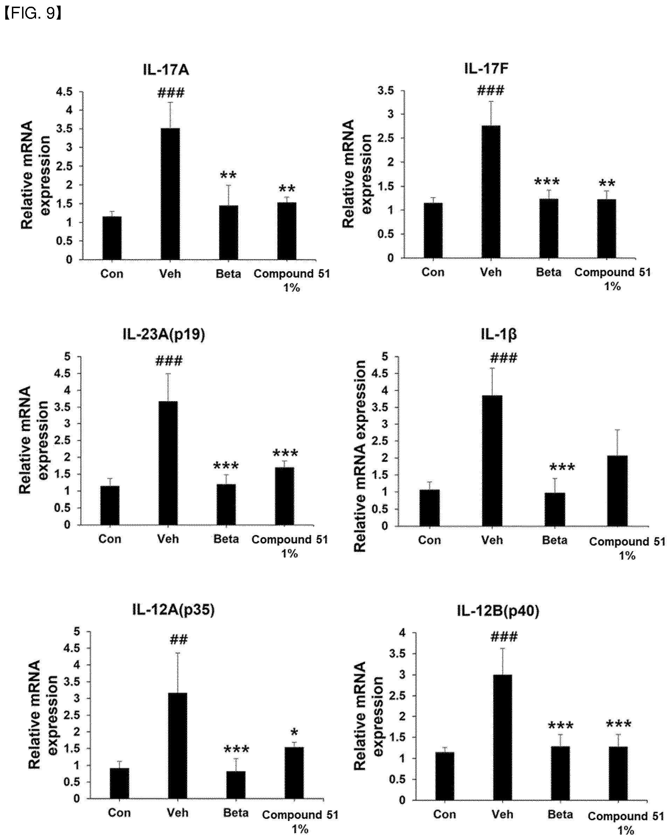

In order to confirm the efficacy of the psoriasis mouse model, the effect of compound 51 of the present invention on gene expression was confirmed using RT-qPCR using primer sets for each specific gene. As a result of examining the psoriasis-related cytokine genes IL-17A, IL-17F, IL-23A(p19), IL-12A(p35), IL-12B(p40), and IL-1β, they were significantly increased in the Veh group. It was confirmed that psoriasis-related cytokine genes were significantly reduced in the Beta group and the Compound 51 treatment group of the present invention. (*p<0.05, **p<0.01, ***p<0.001, Fig. 9).

Tissue was pulverized using a T10 basic ULTRA-TURRAX Homogenizer (IKA, Cat. No. 3737000) into which TRIzol^{™} Reagent (Invitrogen, Cat. No. 15596018) was added. Afterwards, RNA was obtained using isopropyl alcohol (Sigma, Cat.No. 190764) and ethanol (Daejung, Cat.No. 4023-4100), then dissolved in DEPC Water (Biosesang, Cat.No. WR2004-050-00) and stored. After measuring RNA using a spectrophotometer, it was synthesized into cDNA using reverse transcriptase and oligo dT, followed by quantitative RT-PCR. The measurement results were normalized to 18s rRNA, an endogenous reference gene. The primers used for qRT-PCR were as follows: FW-IL-17A: TCTCCACCGCAATGAAGACC (SEQ ID NO.: 1), RV-IL-17A: AAAGTGAAGGGGCAGCTCTC (SEQ ID NO.: 2), FW-IL-17F: TGCTACTGTTGATGTTGGGAC (SEQ ID NO.: 3), RV-IL-17F: AATGCCCTGGTTTTGGTTGAA (SEQ ID NO.: 4), FW-IL-23A(p19): TGGAGCAACTTCACACCTCC (SEQ ID NO.: 5), RV-IL-23A(p19): GGCAGCTATGGCCAAAAAGG (SEQ ID NO.: 6), FW-IL-12A(p35): ACCTGCTGAAGACCACAGATG (SEQ ID NO.: 7), RV-IL-12A(p35): GCTCCCTCTTGTTGTGGAAGA (SEQ ID NO.: 8), FW-IL-12B(p40): CCTGTGACACGCCTGAAGAA (SEQ ID NO.: 9), RV-IL-12B(p40): GTGAGTGGCTCAGAGTCTCG (SEQ ID NO.: 10), FW-IL-1β: CCAAAAGATGAAGGGCTGCTT (SEQ ID NO.: 11), RV-IL-1β: GAAAAGAAGGTGCTCATGTCCTC (SEQ ID NO.: 12), FW-18s rRNA: GTAACCCGTTGAACCCCATT (SEQ ID NO.: 13), RV-18s rRNA: CCATCCAATCGGTAGTAGCG (SEQ ID NO.: 14).

All experimental results obtained in the experiment were expressed as mean value ± standard deviation and graphed using GraphPad Prism software (version 5.01; GraphPad Software, Inc.). When significance was observed by one-way analysis of variance (ANOVA), a post hoc test was performed with Dunnett's multiple comparison method to find out a test group with a significant difference from the control group Veh.

### [Industrial Applicability]

The compounds of the present invention can be used for preventing or treating diseases related to the regulation of Th2 immune response, particularly inflammatory diseases. A composition containing the compound of the present invention may be used for preventing or treating asthma, psoriasis, atopic dermatitis and inflammatory bowel disease, etc.

## Claims

1. A pharmaceutical composition for preventing or treating inflammatory diseases containing a compound of Formula 1, an enantiomer, an optical isomer, a stereoisomer, a diastereomer, a tautomer, or a pharmaceutically acceptable salt thereof as an active ingredient: 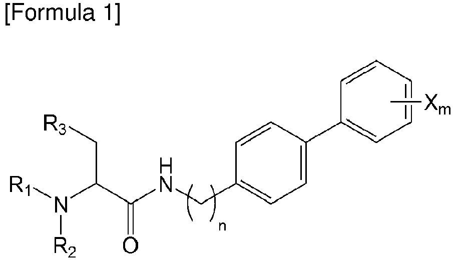
wherein, n is 0, 1 or 2,
R₁ and R₂ are each independently the same as or different from each other, and are each independently H or C₁₋₆ alkyl,
R₃ is C₁₋₆ alkyl, and
X is m substituents (m is an integer from 1 to 5) which are the same as or different from each other, selected from the group consisting of a halogen group, a halogenated C₁₋₆ alkyl group and a halogenated C₁₋₆ alkoxy group.

2. The pharmaceutical composition of claim 1, wherein the inflammatory disease is selected from the group consisting of atopic dermatitis, asthma, allergic rhinitis, allergic conjunctivitis, allergic dermatitis, and psoriasis.

3. A pharmaceutical preparation for preventing or treating fibrosis disease containing the compound of Formula 1, its enantiomer, optical isomer, stereoisomer, diastereomer, tautomer, or pharmaceutically acceptable salt thereof as an active ingredient: 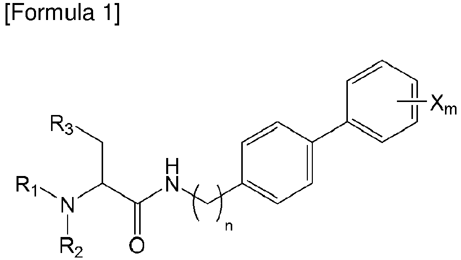
wherein, n is 0, 1 or 2,
R₁ and R₂ are each independently the same as or different from each other, and are each independently H or C₁₋₆ alkyl,
R₃ is C₁₋₆ alkyl, and
X is m substituents (m is an integer from 1 to 5) which are the same as or different from each other, selected from the group consisting of a halogen group, a halogenated C₁₋₆ alkyl group and a halogenated C₁₋₆ alkoxy group.

4. The pharmaceutical composition of claim 3, wherein the fibrosis is one or more selected from the group consisting of liver fibrosis, chronic hepatitis, cirrhosis, hepatic cancer, chemotherapy-associated steatohepatitis (chemotherapy-associated steatohepatitis, CASH), Nonalcoholic steatohepatitis (NASH), lung fibrosis, renal fibrosis, renal failure, pancreatic fibrosis, chronic pancreatitis and pancreatic cancer.

5. The pharmaceutical composition of claim 1 or 2, which inhibits TSLP signaling.

6. The pharmaceutical composition of claim 3 or 4, which inhibits TSLP signaling.

7. A method for preventing or treating inflammatory diseases, comprising administering the pharmaceutical composition of claim 2 to a patient in need of prevention or treatment of inflammatory diseases.

8. A method for preventing or treating fibrotic diseases, comprising administering the pharmaceutical composition of claim 4 to a patient in need of preventing or treating fibrosis diseases.
